(19) Europäisches Patentamt
European Patent Office
Office européen des brevets

(11) EP 4 563 151 A1

(12) EUROPEAN PATENT APPLICATION
published in accordance with Art. 153(4) EPC

(43) Date of publication:
**04.06.2025 Bulletin 2025/23**

(21) Application number: **23845711.3**

(22) Date of filing: **28.07.2023**

(51) International Patent Classification (IPC):
***A61K 31/553*** *(2006.01)* ***A61K 9/127*** *(2025.01)*
***A61P 35/00*** *(2006.01)*

(52) Cooperative Patent Classification (CPC):
**A61K 9/127; A61K 31/553; A61P 35/00**

(86) International application number:
**PCT/CN2023/109901**

(87) International publication number:
**WO 2024/022507 (01.02.2024 Gazette 2024/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.07.2022 CN 202210909959**
**20.12.2022 CN 202211642769**

(71) Applicants:
- **Jiangsu Hengrui Pharmaceuticals Co., Ltd.
Lianyungang, Jiangsu 222047 (CN)**
- **Shanghai Hengrui Pharmaceutical Co., Ltd.
Shanghai 200245 (CN)**

(72) Inventors:
- **SHA, Ruilin
Shanghai 200245 (CN)**
- **DUAN, Ziqing
Shanghai 200245 (CN)**
- **WANG, Haifeng
Shanghai 200245 (CN)**
- **TONG, Xinyong
Shanghai 200245 (CN)**
- **WANG, Pingping
Shanghai 200245 (CN)**
- **FENG, Biao
Shanghai 200245 (CN)**
- **WANG, Bin
Shanghai 200245 (CN)**

(74) Representative: **Regimbeau
20, rue de Chazelles
75847 Paris Cedex 17 (FR)**

# (54) PHARMACEUTICAL COMPOSITION COMPRISING KRAS G12D INHIBITOR

(57) The present disclosure relates to a pharmaceutical composition comprising a KRAS G12D inhibitor. In particular, the present disclosure relates to a pharmaceutical composition, comprising a compound represented by formula I or a pharmaceutically acceptable salt thereof, and a lipid. The pharmaceutical composition can be better applied clinically.

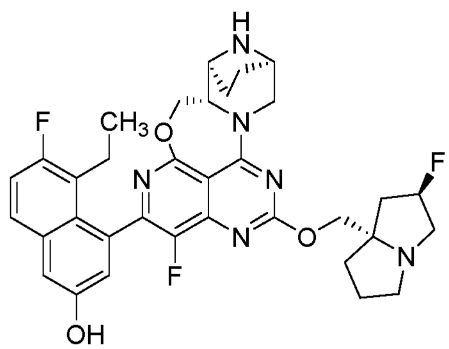

(I)

EP 4 563 151 A1

## Description

### TECHNICAL FIELD

The present disclosure pertains to the field of pharmaceutical formulations, and particularly relates to a pharmaceutical composition comprising a KRAS G12D inhibitor and a preparation method therefor.

### BACKGROUND

RAS is one of the oncogenes with the highest mutation rate in tumors, and about 30% of human malignancies are associated with mutations of the RAS gene. The RAS family includes KRAS, NRAS, and HRAS, and KRAS mutations are the most common and account for approximately 85%. KRAS mutations are common in solid tumors, with highfrequency mutations in the three fatal cancers in humans: lung cancer (17%), colorectal cancer (33%), and pancreatic cancer (61%). A total of 97% of the KRAS gene mutations involve mutation of amino acid residue No. 12 or 13, and G12D is an important mutation. Data analysis on the European and American population shows that G12D mutation is found in 36%, 12%, and 4% of pancreatic cancer, colorectal cancer, and non-small cell lung cancer patients, respectively.

After KRAS is activated, it regulates multiple functions such as cell proliferation, survival, migration, and metabolism through a plurality of downstream signaling pathways represented by RAF-MEK-ERK, PI3K-AKT-mTOR, and TIAM1-RAc. After mutation of the KRAS gene, the protein is continuously activated, resulting in continuous activation of downstream signaling pathways and thereby promoting tumorigenesis.

KRAS protein has long been considered as an undruggable drug target because it lacks conventional small molecule binding sites on its surface and it is extremely difficult to inhibit due to its ultrahigh affinity for guanylic acid. However, based on the importance and prevalence of abnormal KRAS activation in cancer progression, KRAS has been and remains a target of high interest for drug development. Currently, except for inhibitors against KRAS G12C, there is a lack of KRAS inhibitors that are effective against other mutations, such that most patients with KRAS mutation remain non-medicated. G12D is a mutant widely expressed in multiple tumors, and it is of important clinical significance to develop inhibitors against it.

WO2022268051 provides a novel KRAS G12D inhibitor compound (formula I) that has relatively good pharmaceutical activity.

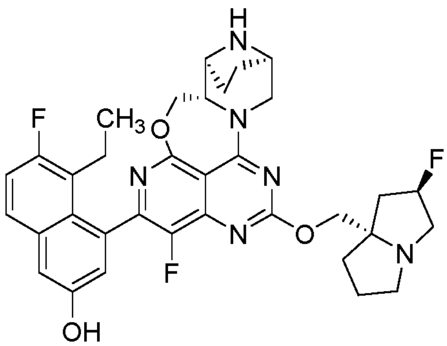

(I)

### SUMMARY

The present disclosure aims to provide a pharmaceutical composition comprising a compound of formula I or a pharmaceutically acceptable salt thereof.

In one aspect, the present disclosure provides a pharmaceutical composition comprising a compound of formula I or a pharmaceutically acceptable salt thereof and a lipid,

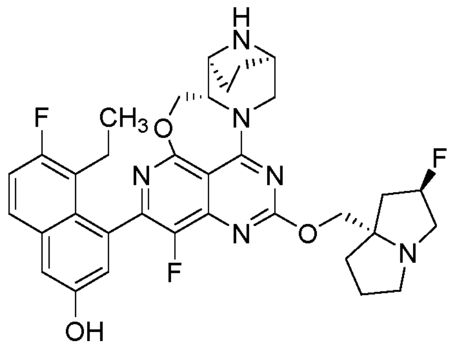

(I)

In some embodiments, the lipid comprises at least one phospholipid.

In some embodiments, the pharmaceutical composition of the present disclosure is a liposome.

In another aspect, the present disclosure provides a liposome comprising a compound of formula I or a pharmaceutically acceptable salt thereof.

The lipid of the present disclosure may include completely neutral or negatively charged phospholipids. The term "phospholipid" refers to a hydrophobic molecule containing at least one phosphorus group, which may be natural or synthetic. For example, a phospholipid may comprise a phosphorus-containing group and a saturated or unsaturated alkyl group optionally substituted with OH, COOH, oxo, amine, or a substituted or unsubstituted aryl group. Phospholipids differ from each other in the length and the degree of unsaturation of their acyclic chains.

In some embodiments, the phospholipid includes one or more of phosphatidylcholine, phosphatidylethanolamine, phosphatidylglycerol, phosphatidylserine, phosphatidic acid, and phosphatidylinositol. The term "phosphatidylcholine" refers to phosphatidylcholine and derivatives thereof. Examples of phospholipids suitable for use in the present disclosure include dipalmitoylphosphatidylcholine (DPPC), distearoylphosphatidylcholine (DSPC), dimyristoylphosphatidylcholine (DMPC), 1-palmitoyl-2-linoleoyl-sn-glycero-3-phosphatidylcholine (PLPC), dioleoylphosphatidylcholine (DOPC), dierucoylphosphatidylcholine (DEPC), egg yolk phosphatidylcholine (EPC), dilauroylphosphatidylcholine (DLPC), hydrogenated soy phosphatidylcholine (HSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoyl phosphatidylcholine (SPPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), dioleoylphosphatidylglycerol (DOPG), dimyristoylphosphatidylglycerol (DMPG), distearoylphosphatidylglycerol (DSPG), dipalmitoylglycerophosphoglycerol (DPPG), dipalmitoylphosphatidylserine (DPPS), 1,2-dioleoyl-sn-glycero-3-phosphatidylserine (DOPS), dimyristoylphosphatidylserine (DMPS), distearoylphosphatidylserine (DSPS), dipalmitoylphosphatidic acid (DPPA), 1,2-dioleoyl-sn-glycero-3-phosphatidic acid (DOPA), dimyristoylphosphatidic acid (DMPA), distearoylphosphatidic acid (DSPA), dipalmitoylphosphatidylinositol (DPPI), 1,2-dioleoyl-sn-glycero-3-phosphatidylinositol (DOPI), dimyristoylphosphatidylinositol (DMPI), distearoylphosphatidylinositol (DSPI), and derivatives (e.g., hydrophilic polymer-modified derivatives) thereof.

The hydrophilic polymer-modified phospholipids include, but are not limited to, polyethylene glycol-modified distearoylphosphatidylethanolamine (PEG-DSPE), polyethylene glycol-modified distearoyl phosphatidylglycerol (PEG-DSPG), and polyethylene glycol-modified cholesterol (PEG-CHOL). PEG has a molecular weight of 200-10,000 Da, for example, 1000-5000 Da, or 2000-5000 Da.

In some embodiments, the phospholipid in the present disclosure is selected from the group consisting of dipalmitoylphosphatidylcholine (DPPC), distearoylphosphatidylcholine (DSPC), dioleoylphosphatidylcholine (DOPC), dierucoylphosphatidylcholine (DEPC), dilauroylphosphatidylcholine (DLPC), hydrogenated soy phosphatidylcholine (HSPC), distearoylphosphatidylethanolamine (DSPE), and dimyristoylphosphatidylcholine (DMPC), and polyethylene glycol-modified derivatives thereof.

In some embodiments, the liposome comprises polyethylene glycol-modified distearoylphosphatidylethanolamine. In some embodiments, the polyethylene glycol in the polyethylene glycol-modified distearoylphosphatidylethanolamine has a molecular weight of 200-10,000 Da. In some embodiments, the polyethylene glycol in the polyethylene glycol-modified distearoylphosphatidylethanolamine has a molecular weight of 1000-5000 Da. In some embodiments, the polyethylene glycol in the polyethylene glycol-modified distearoylphosphatidylethanolamine has a molecular weight of 2000-5000 Da.

In some embodiments, the polyethylene glycol-modified distearoylphosphatidylethanolamine is selected from the group consisting of polyethylene glycol 2000-distearoylphosphatidylethanolamine (mPEG2000-DSPE).

In some embodiments, the phospholipid has a molar content selected from the group consisting of 10-80%, including but not limited to 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, or any value between any two of these values, for example, 30-70%, relative to the total amount of the lipid mixture.

The present disclosure may also comprise other neutral, cationic, and/or anionic lipids.

Examples of other neutral lipids that can be used in the present disclosure include one or more of steroids such as cholesterol and derivatives thereof, lecithin, soy phospholipid, cephalin, sphingomyelin, and hydrogenated soy phospholipid. In some embodiments, the molar percentage of a steroid does not exceed 90%, relative to the total amount of the lipid mixture.

In some embodiments, the lipid comprises at least one phospholipid and cholesterol. The molar percentage of cholesterol may be 0.1%-90%, for example, 10%-80% or 20%-70%, relative to the total amount of the lipid mixture.

In some embodiments, the phospholipid is selected from the group consisting of dipalmitoylphosphatidylcholine (DPPC), distearoylphosphatidylcholine (DSPC), dioleoylphosphatidylcholine (DOPC), dierucoylphosphatidylcholine (DEPC), dilauroylphosphatidylcholine (DLPC), hydrogenated soy phosphatidylcholine (HSPC), distearoylphosphatidylethanolamine (DSPE), and dimyristoylphosphatidylcholine (DMPC), and polyethylene glycol-modified derivatives thereof.

In some embodiments, the lipid comprises hydrogenated soy phosphatidylcholine, cholesterol, and polyethylene glycol 2000-distearoylphosphatidylethanolamine.

In some embodiments, the hydrophilic polymer-modified phospholipid derivative has a content (relative to all liposome membrane components, mole fraction) selected from the group consisting of 0.1-50%, including but not limited to 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, or any value between any two of these values; for example, the content may be 0.5-20%.

In some embodiments, the pharmaceutical composition further comprises a trapping agent. In some embodiments, an inner aqueous phase of the liposome comprises a trapping agent.

The liposome may be prepared by active drug loading. Active drug loading is a drug loading method for transporting a therapeutic agent from an external medium across the bilayer membrane of the liposome to an internal aqueous space via a polyatomic ion-gradient. These gradients are generated by embedding at least one polyatomic ion as a trapping agent in the internal aqueous space of the liposome and replacing the external medium of the liposome with an additional medium having a relatively low concentration of polyatomic ions, such as, pure water, sucrose solution, or normal saline, by known techniques such as column separation, dialysis, or centrifugation. A polyatomic ion gradient is created between the internal aqueous space and the external medium of the liposome to trap the therapeutic agent in the internal aqueous space of the liposome.

The trapping agent of the present disclosure includes, but is not limited to, a sulfate, a sulfite, a gluconate, a phosphate, a hydrogen phosphate, a molybdate, a carbonate, a nitrate, and the like. Exemplary trapping agents include, but are not limited to, ammonium sulfate, ammonium phosphate, ammonium molybdate, ammonium sucrose octasulfate, triethylammonium sucrose octasulfate, copper gluconate, ethylammonium sulfate, ammonium ethylenediaminetetraacetate, ammonium chloride, ammonium hydroxide, ammonium acetate, dextran ammonium sulfate, or triethyldextran ammonium sulfate, and combinations thereof.

In some embodiments, the trapping agent is ammonium sulfate or copper gluconate.

In some embodiments, the trapping agent in the inner aqueous phase of the liposome has a concentration selected from the group consisting of 10-400 mM, which may be 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 155 mM, 160 mM, 165 mM, 170 mM, 175 mM, 180 mM, 185 mM, 190 mM, 195 mM, 200 mM, 205 mM, 210 mM, 215 mM, 220 mM, 225 mM, 230 mM, 235 mM, 240 mM, 245 mM, 250 mM, 255 mM, 260 mM, 265 mM, 270 mM, 275 mM, 280 mM, 285 mM, 290 mM, 295 mM, 300 mM, 305 mM, 310 mM, 315 mM, 320 mM, 325 mM, 330 mM, 335 mM, 340 mM, 345 mM, 350 mM, 355 mM, 360 mM, 365 mM, 370 mM, 375 mM, 380 mM, 385 mM, 390 mM, 395 mM, 400 mM, or any value between any two of these values, for example, 20-400 mM.

In some embodiments, the pharmaceutical composition is a liposome comprising a compound of formula I or a pharmaceutically acceptable salt thereof, a lipid, and a trapping agent, wherein the lipid comprises at least one phospholipid and a steroid.

In some embodiments, the phospholipid is selected from the group consisting of dipalmitoylphosphatidylcholine (DPPC), distearoylphosphatidylcholine (DSPC), dioleoylphosphatidylcholine (DOPC), dierucoylphosphatidylcholine (DEPC), dilauroylphosphatidylcholine (DLPC), hydrogenated soy phosphatidylcholine (HSPC), distearoylphosphatidylethanolamine (DSPE), and dimyristoylphosphatidylcholine (DMPC), and polyethylene glycol-modified derivatives thereof; the steroid is cholesterol. In some embodiments, the inner aqueous phase of the liposome of the present disclosure further comprises at least one pH adjuster, and the pH adjuster may be selected from the group consisting of an amino acid such as arginine, histidine, or glycine; an acid such as ethylenediaminetetraacetic acid, pentetic acid, hydrochloric acid, phosphoric acid, malic acid, or sulfuric acid; a salt such as a sodium salt, a potassium salt, or an ammonium salt of the aforementioned acid; a basic compound (alkali) such as ammonia water, sodium hydroxide, potassium hydroxide, or triethanolamine; and the like; for example, the pH adjuster may be sodium hydroxide, hydrochloric acid, ammonia water, phosphoric acid, malic acid, histidine, or triethanolamine. In addition, the pH adjuster may be used in combination with two or more of the aforementioned ammonium salts.

In some embodiments, the pH adjuster in the inner aqueous phase of the liposome has a concentration selected from the group consisting of 1-300 mM, which may be 1 mM, 2 mM, 3 mM, 4 mM, 5 mM, 6 mM, 7 mM, 8 mM, 9 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 155 mM, 160 mM, 165 mM, 170 mM, 175 mM, 180 mM, 185 mM, 190 mM, 195 mM, 200 mM, 205 mM, 210 mM, 215 mM, 220 mM, 225 mM, 230 mM, 235 mM, 240 mM, 245 mM, 250 mM, 255 mM, 260 mM, 265 mM, 270 mM, 275 mM, 280 mM, 285 mM, 290 mM, 295 mM, 300 mM, or any value between any two of these values, for example, 20-300 mM.

In some embodiments, the compound of formula I or the pharmaceutically acceptable salt thereof has a concentration selected from the group consisting of 0.01-100 mg/mL, which may be 0.01 mg/mL, 0.05 mg/mL, 0.10 mg/mL, 0.15 mg/mL, 0.20 mg/mL, 0.25 mg/mL, 0.30 mg/mL, 0.35 mg/mL, 0.40 mg/mL, 0.45 mg/mL, 0.50 mg/mL, 0.55 mg/mL, 0.60 mg/mL, 0.65 mg/mL, 0.70 mg/mL, 0.75 mg/mL, 0.80 mg/mL, 0.85 mg/mL, 0.90 mg/mL, 0.95 mg/mL, 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL or any value between any two of these values, for example, 0.05-10 mg/mL.

In another aspect, in order to improve the encapsulation efficiency of the compound of formula I or the pharmaceutically acceptable salt thereof in the liposome, a solution containing an electrolyte (salt solution) may be added to the outer aqueous phase of the liposome to increase the ionic strength, thereby increasing the encapsulation efficiency. The electrolyte (salt) contained in the outer aqueous phase of the liposome is not particularly limited, and may be, for example, sodium chloride or potassium chloride, for example, sodium chloride. In addition, normal saline may also be used. In addition, the outer aqueous phase of the liposome as a liposome dispersion or the like may contain a sugar, an electrolyte, and/or an amino acid or a salt thereof, or may contain a sugar or an electrolyte, and an amino acid or a salt thereof. As an example, the sugar is selected from the group consisting of sucrose, trehalose, and glucose, the electrolyte is selected from the group consisting of normal saline, sodium chloride, and potassium chloride, and the amino acid or the salt thereof may be selected from the group consisting of histidine and a salt thereof.

In some embodiments, the outer aqueous phase of the liposome comprises sodium chloride and histidine or a salt thereof.

In some embodiments, the amino acid in the outer aqueous phase of the liposome has a concentration selected from the group consisting of 1-300 mM, which may be 1 mM, 2 mM, 3 mM, 4 mM, 5 mM, 6 mM, 7 mM, 8 mM, 9 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 155 mM, 160 mM, 165 mM, 170 mM, 175 mM, 180 mM, 185 mM, 190 mM, 195 mM, 200 mM, 205 mM, 210 mM, 215 mM, 220 mM, 225 mM, 230 mM, 235 mM, 240 mM, 245 mM, 250 mM, 255 mM, 260 mM, 265 mM, 270 mM, 275 mM, 280 mM, 285 mM, 290 mM, 295 mM, 300 mM, or any value between any two of these values, for example, 5-200 mM.

In some embodiments, the pharmaceutical composition is a liposome comprising a compound of formula I or a pharmaceutically acceptable salt thereof and a lipid, wherein the lipid comprises at least one phospholipid and a steroid. An inner aqueous phase of the liposome comprises a trapping agent and a pH adjuster, and an outer aqueous phase of the liposome comprises one or more selected from the group consisting of a sugar, an electrolyte, and an amino acid or a salt thereof.

In some embodiments, the inner aqueous phase of the liposome of the present disclosure has a pH of 5-8; the outer aqueous phase has a pH of 5-11. Preferably, the inner aqueous phase has a pH of 5-8; the outer aqueous phase has a pH of 5-8.

The present disclosure also provides a method for preparing a liposome, comprising the steps of preparing a blank liposome, and introducing a compound of formula I or a pharmaceutically acceptable salt thereof into an inner aqueous phase of the blank liposome.

In the present disclosure, the blank liposome may be prepared using a film dispersion method, a reverse-phase evaporation method, a direct hydration method, a surfactant removing method, an ethanol injection method, and a modified ethanol injection method. In some embodiments, a blank liposome with the required particle size may be obtained by processing with a probe sonicator, a high-pressure homogenizer, a microfluidizer, an extruder, and other apparatus.

In addition, various parameters (e.g., the amount of membrane components, temperature, etc.) in the liposome

preparation process can be appropriately adjusted and determined according to the method for preparing the liposome or the composition, particle size and the like of the liposome of interest.

In another aspect, the replacement or dilution operation of the present disclosure may be performed by dialysis, centrifugal separation, gel filtration, and other methods. In addition, the active substance may also be effectively encapsulated in the inner aqueous phase of the liposome by replacing or diluting the outer aqueous phase of the liposome.

Dialysis, centrifugal separation, and gel filtration are all conventional procedures in the art. For example, dialysis may be performed using a dialysis membrane. The dialysis membrane may be made of polyethersulfone.

In some embodiments, the pharmaceutical composition is a liquid formulation, and the liposome composition obtained from the above introduction step may be directly used as a final liposome composition (pharmaceutical composition), or the outer aqueous phase of the liposome of the resulting liposome composition may be adjusted (replaced, etc.) to give a final liposome composition (pharmaceutical composition).

In some other embodiments, the pharmaceutical composition is a solid formulation, and the liposome composition obtained from the above introduction step may be dried and used as a final solid liposome composition (pharmaceutical composition). The method for drying the liposome composition may include, but is not limited to, lyophilization, spray drying, and the like.

In another aspect, the pharmaceutical composition is a solid formulation, and the pharmaceutical composition may be dissolved and suspended in an appropriate solvent and used as a liquid formulation. The solvent may be appropriately set according to the purpose of use of the liposome composition. For example, when the pharmaceutical composition is used as an injection, the solvent is preferably sterilized distilled water.

The present disclosure also provides a lyophilized composition obtained by lyophilizing the aforementioned pharmaceutical composition, or the lyophilized composition is reconstituted in a liquid medium to give the aforementioned pharmaceutical composition, wherein the liquid medium used for the reconstitution is selected from the group consisting of normal saline, water for injection, a glucose injection, and a glucose and sodium chloride injection.

The present disclosure further provides use of the aforementioned pharmaceutical composition, liposome, lyophilized composition, or reconstituted solution in the preparation of a medicament for treating and/or preventing a disease or disorder, wherein the disease or disorder is a cancer.

The disease or disorder in the present disclosure is selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, renal cancer, pancreatic cancer, gallbladder cancer, bile duct cancer, colorectal cancer, small intestine cancer, gastrointestinal stromal tumor, urothelial cancer, urinary tract cancer, bladder cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, lipoma, bone cancer, soft tissue sarcoma, neurofibroma, neuroglioma, neuroblastoma, and glioblastoma.

In some embodiments, the disease or disorder is selected from the group consisting of pancreatic cancer, colorectal cancer, and non-small cell lung cancer.

The pharmaceutical composition of the present disclosure has good stability, high liposome encapsulation efficiency, large drug loading capacity, and low liposome-related allergy, and can be better applied clinically.

As used herein, the term "mass/volume percentage" (w/v) refers to the mass (g) of an ingredient contained in every 100 mL of a liquid system, i.e., g/100 mL.

The contents (including percentage contents) of the various substances of the present disclosure and their ratios to each other are allowed to have an error of $\pm 5\%$. For example, "the content of the compound of formula I or the pharmaceutically acceptable salt thereof in the composition is 5-20 mg/mL" means that the content of the compound of formula I or the pharmaceutically acceptable salt thereof in the composition being 4.75-21 mg/mL falls within the scope of the present disclosure; "the weight ratio of the compound of the formula I or the pharmaceutically acceptable salt thereof to the lipid is selected from the group consisting of 1:1-1:100" means that the weight ratio of the compound of the formula I or the pharmaceutically acceptable salt thereof to the lipid being selected from the group consisting of 1:0.95-1:105 falls within the scope of the present disclosure.

As used herein, the terms "mixed into" and "mixed" mean that the order of addition of the components is not limited. For example, when A is mixed into B, it can mean either A is added to B or B is added to A; when A and B are mixed, it can mean either A is added to B and mixed or B is added to A and mixed.

Liposome refers to microscopic lipid vesicles composed of a bilayer phospholipid or any similar amphiphilic lipid encapsulating an inner aqueous medium. The liposome of the present disclosure may be multilamellar vesicles (MLVs), large unilamellar vesicles (LUVs), or small unilamellar vesicles, with the size usually ranging from 30 nm-200 nm. In addition, there is no specific limitation on the liposome membrane structure in the present disclosure.

Liposome membrane refers to a bilayer phospholipid that separates the inner aqueous medium from the outer aqueous medium.

Inner aqueous phase of liposome refers to an aqueous region surrounded by a lipid bilayer of a liposome, and is used in the same sense as the "inner aqueous phase" and the "liposomal inner aqueous phase". When the liposome is

dispersed in a liquid, "outer aqueous phase of the liposome" refers to a region not surrounded by a lipid bilayer of a liposome (i.e., a region excluding the inner aqueous phase and the lipid bilayer).

Blank liposome refers to a liposome free of the active ingredient to be loaded, such as the compound of formula I of the present disclosure, in the inner aqueous phase.

Encapsulation efficiency refers to the percentage of an encapsulated substance (such as a drug) in a liposome suspension relative to the total amount of the drug. It is an important index for controlling the quality of liposomes and nanoparticles, and reflects the degree of encapsulation of the drug by a carrier.

The encapsulation efficiency includes the determination of the encapsulation percentage and the encapsulation volume, and in general, the encapsulation percentage (EN%) is mainly investigated in the literature. The expression is EN% = (1 - Cf/Ct) × 100%. In the expression, Cf is the amount of free drug; Ct is the total amount of drug in the liposome suspension.

$$\text{Encapsulation efficiency (EN \%)} = 1 - \frac{\text{Amount of free drug}}{\text{Total amount of drug in the liposome suspension}} \times 100\%$$

The encapsulation efficiency can be determined by conventional determination methods for encapsulation efficiency, such as HPLC.

As used herein, "polymer molecular weight" is taken as number-average molecular weight and refers to the statistical average of the molecular weights of all polymeric chains in a sample, which is defined as follows, where $M_i$ represents the molecular weight of a single chain and $N_i$ represents the number of chains with the corresponding molecular weight. $M_n$ can be predicted by the polymerization mechanism and determined by a method for measuring the number of molecules in a sample of a given mass, e.g., a colligative method such as terminal analysis. If $M_n$ is used to characterize the molecular weight distribution, there are an equal number of molecules distributed on both sides of $M_n$.

The term "particle size distribution" or "PSD" refers to the particle size distribution in the liposome composition as measured by the dynamic light scattering technique well known to those skilled in the art, for example, using the Malvern Mastersizer™ 2000. As used herein, "d (0.1)" refers to the particle size at which the cumulative particle size distribution percentage of a sample reaches 10%. "d(0.5)" refers to the particle size at which the cumulative particle size distribution percentage of a sample reaches 50%. "d(0.9)" refers to the particle size at which the cumulative particle size distribution percentage of a sample reaches 90%.

## BRIEF DESCRIPTION OF THE DRAWINGS

FIG. 1 shows a cryo-scanning electron microscope (Cryo-TEM) image of the liposome of Example 5;
FIG. 2 shows a cryo-scanning electron microscope (Cryo-TEM) image of the liposome of Example 6.

## DETAILED DESCRIPTION

The present disclosure is further illustrated in detail by the following examples. These examples are for illustrative purposes only and are not intended to limit the scope of the present disclosure. PCT/CN2022/100016 is incorporated into the present disclosure by reference in its entirety.

The structures of the compounds were determined by nuclear magnetic resonance (NMR) spectroscopy or/and mass spectrometry (MS). The NMR shifts (δ) are given in $10^{-6}$ (ppm). The NMR analyses were performed on a Bruker AVANCE-400 nuclear magnetic resonance instrument or Bruker AVANCE NEO 500M, with dimethyl sulfoxide-D6 (DMSO-$d_6$), chloroform-D ($CDCl_3$), and methanol-D4 ($CD_3OD$) as solvents and tetramethylsilane (TMS) as an internal standard.

The MS analyses were performed on an Agilent 1200/1290 DAD-6110/6120 Quadrupole MS liquid chromatography-mass spectrometry system (manufacturer: Agilent; MS model: 6110/6120 Quadrupole MS),

waters ACQuity UPLC-QD/SQD (manufacturer: waters; MS model: waters ACQuity Qda Detector/waters SQ Detector), and
THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and Waters HPLC e2695-2489 high-pressure liquid chromatographs.

The chiral HPLC analyses were performed using an Agilent 1260 DAD high performance liquid chromatograph.

The preparative high performance liquid chromatography steps were performed using Waters 2545-2767,

Waters 2767-SQ Detecor2, Shimadzu LC-20AP, and Gilson GX-281 preparative chromatographs.

The preparative chiral chromatography steps were performed using a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

In the silica gel column chromatography, a 200-300 mesh silica gel (Huanghai, Yantai) was generally used as the carrier.

The kinase mean inhibition rates and $IC_{50}$ values were measured using a NovoStar microplate reader (BMG, Germany).

Known starting materials described herein may be synthesized by using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 rounds of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of the reaction progress in the examples was conducted by thin-layer chromatography (TLC). The developing solvent used in the reactions, the eluent systems used in the column chromatography purification, and the developing solvent systems used in the thin-layer chromatography analyses include: A: a dichloromethane/methanol system, and B: n-hexane/ethyl acetate. The volume ratio of the solvents was adjusted according to the polarity of the compound, or by adding a small amount of basic or acidic reagents such as triethylamine and acetic acid.

When the compounds of the examples contained two or more chiral centers, the relative stereochemistry of these compounds was identified by NMR studies and/or X-ray diffraction. In these cases, the compounds were identified using the prefix "rel" followed by the R/S nomenclature, where R/S provides only relative stereochemical information and does not indicate absolute stereochemistry. For example,

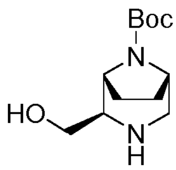

(±)-*rel-(1R,2R,5S)*

1g

represents a 1:1 mixture of

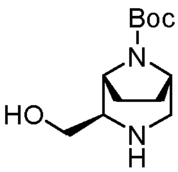

*(1R,2R,5S)* and

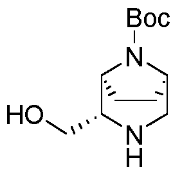

*(1S,2S,5R)*,

i.e., a racemate.

## Example 1

*tert*-Butyl (±)-*rel*-(1*R*,2*R*,5*S*)-2-(hydroxymethyl)-3,8-diazabicyclo[3.2.1]octane-8-carboxylate **1g**

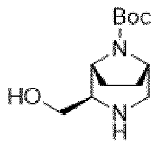

(±)-*rel*-(1R,2R,5S))

1g

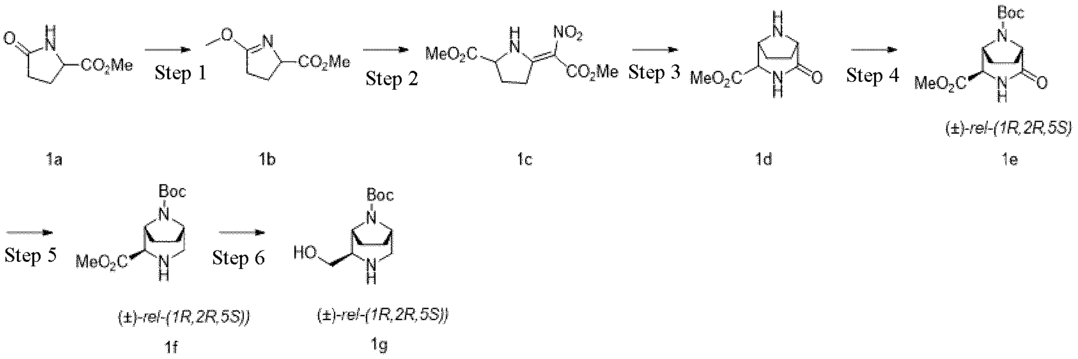


Step 1

Methyl (±)-5-methoxy-3,4-dihydro-2*H*-pyrrole-2-carboxylate **1b**

Methyl (±)-2-pyrrolidone-5-formate **1a** (100 g, 698.61 mmol, Shanghai Bide) and dimethyl sulfate (110 g, 872.10 mmol) were mixed and reacted at 60 °C for 16 h, and the reaction mixture was cooled to room temperature, and poured into a solution of triethylamine (100 g) and methyl *tert*-butyl ether (150 mL) under an ice bath. The resulting mixture was extracted with methyl *tert*-butyl ether (300 mL × 6), and then concentration was performed under reduced pressure to give crude title compound **1b** (90 g, yield: 81.9%), which was directly used in the next step without purification.

MS m/z (ESI): 158.1[M+1].

Step 2

Methyl (±)-5-(2-methoxy-1-nitro-2-oxoethylidene)pyrrolidine-2-carboxylate **1c**

Crude compound **1b** (90 g, 572.64 mmol) and methyl nitroacetate (68.18 g, 572.63 mmol) were mixed, heated to 60 °C, and stirred for 30 h. The reaction mixture was cooled to room temperature, and ethyl acetate (300 mL) was then added. The mixture was stirred for 0.5 h and then filtered, and the filter cake was dried to give the title compound **1c** (70 g, yield: 50%), which was directly used in the next step without purification.

MS m/z (ESI): 245.1[M+1].

Step 3

Methyl 4-oxo-3,8-diazabicyclo[3.2.1]octane-2-carboxylate (diastereomeric mixture) **1d**

Crude compound **1c** (14 g, 57.3 mmol) was dissolved in 600 mL of methanol, and a 10% palladium on carbon catalyst (wet) (14 g) was added. The system was purged with hydrogen three times and stirred for 48 h. The reaction mixture was filtered through diatomaceous earth, and the filtrate was concentrated to give crude title compound **1d** (10 g, yield: 94.6%), which was directly used in the next step without purification.

MS m/z (ESI): 185.2[M+1].

Step 4

8-(*tert*-Butyl) 2-methyl (+)-*rel*-(1*R*,2*R*,5*S*)-4-oxo-3,8-diazabicyclo[3.2.1]octane-2,8-diformate **1e**

Crude compound **1d** (10 g, 54.2 mmol) was dissolved in 300 mL of dichloromethane, and triethylamine (16 g, 158.12 mmol) and di-*tert*-butyl dicarbonate (11 g, 50.4 mmol, Shanghai Accela) were added under an ice bath. The mixture was stirred for 14 h and concentrated under reduced pressure, and the residue was purified by silica gel column chromatography with eluent system B to give title compound **1e** (3.3 g, yield: 21.3%). MS m/z (ESI): 285.2 [M+1].
HPLC analysis: retention time: 1.02 min; purity: 98.5% (chromatography column: ACQUITY UPLC®BEH, C18, 1.7 μm, 2.1 × 50 mm; mobile phase: water (10 mM ammonium bicarbonate), acetonitrile; gradient ratio: acetonitrile 10%-95%).

Step 5

8-(*tert*-Butyl) 2-methyl (±)-*rel*-(1*R*,2*R*,5*S*)-3,8-diazabicyclo[3.2.1]octane-2,8-diformate **1f**

Compound **1e** (400 mg, 1.4 mmol) was dissolved in 2 mL of tetrahydrofuran, and 3.5 mL of a 2 M solution of borane dimethylsulfide complex in tetrahydrofuran was added. The mixture was stirred for 14 h, quenched with methanol, reacted at 50 °C for another 14 h, and concentrated under reduced pressure, and the residue was purified by silica gel column chromatography with eluent system A to give title compound **1f** (176 mg, yield: 46.2%).
MS m/z (ESI): 271.2[M+1].

Step 6

*tert*-Butyl (±)-*rel*-(1*R*,2*R*,5*S*)-2-(hydroxymethyl)-3,8-diazabicyclo[3.2.1]octane-8-carboxylate **1g**

Compound **1f** (1 g, 3.69 μmol) was dissolved in 15 mL of tetrahydrofuran, and 4.4 mL of a 1 M solution of lithium aluminum hydride in tetrahydrofuran was added. The mixture was stirred at 0 °C for 1 h. 0.2 mL of water, 0.2 mL of a 15% aqueous sodium hydroxide solution, and 0.4 mL of water were sequentially added, and anhydrous sodium sulfate was added. The mixture was stirred for 10 min and filtered, and the filtrate was concentrated to give title compound **1g** (430 mg, yield: 47.9%), which was directly used in the next step without purification.
MS m/z (ESI): 243.1[M+1].

**Example 2**

2,6-Dichloro-3-fluoropyridin-4-amine **2a**

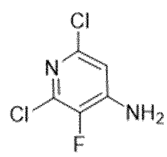

2a

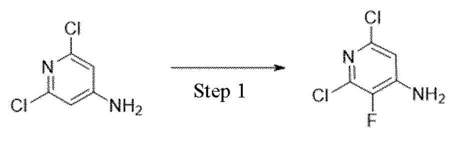


1h 2a

Compound **1h** (5 g, 30.6 mmol) was dissolved in 20 mL of *N,N*-dimethylformamide and 20 mL of acetonitrile, and 1-chloromethyl-4-fluoro-1,4-diazabicyclo[2.2.2]octane bis(tetrafluoroborate) (13 g, 36.8 mmol) was added. The mixture was reacted at 80 °C for 0.5 h and concentrated under reduced pressure, and the residue was purified by silica gel column chromatography with eluent system B to give title compound **3a** (2.2 g, yield: 39.6%).

MS m/z (ESI): 180.9[M+1].

**Example 3**

5-Ethy1-4-((5a*S*,6*S*,9*R*)-1-fluoro-12-(((2*R*,7a*S*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **3-p1**

5-Ethyl-4-((5a*R*,6*R*,9*S*)-1-fluoro-12-(((2R,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **3-p2**

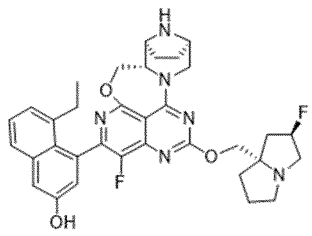

3-p1

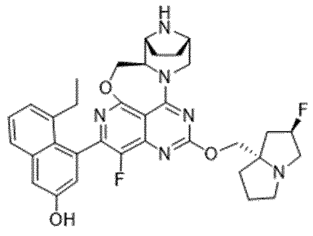

3-p2

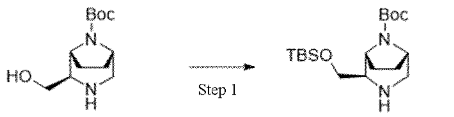


(±)-*rel*-(1*R*,2*R*,5*S*)
1g

(±)-*rel*-(1*R*,2*R*,5*S*)
3a

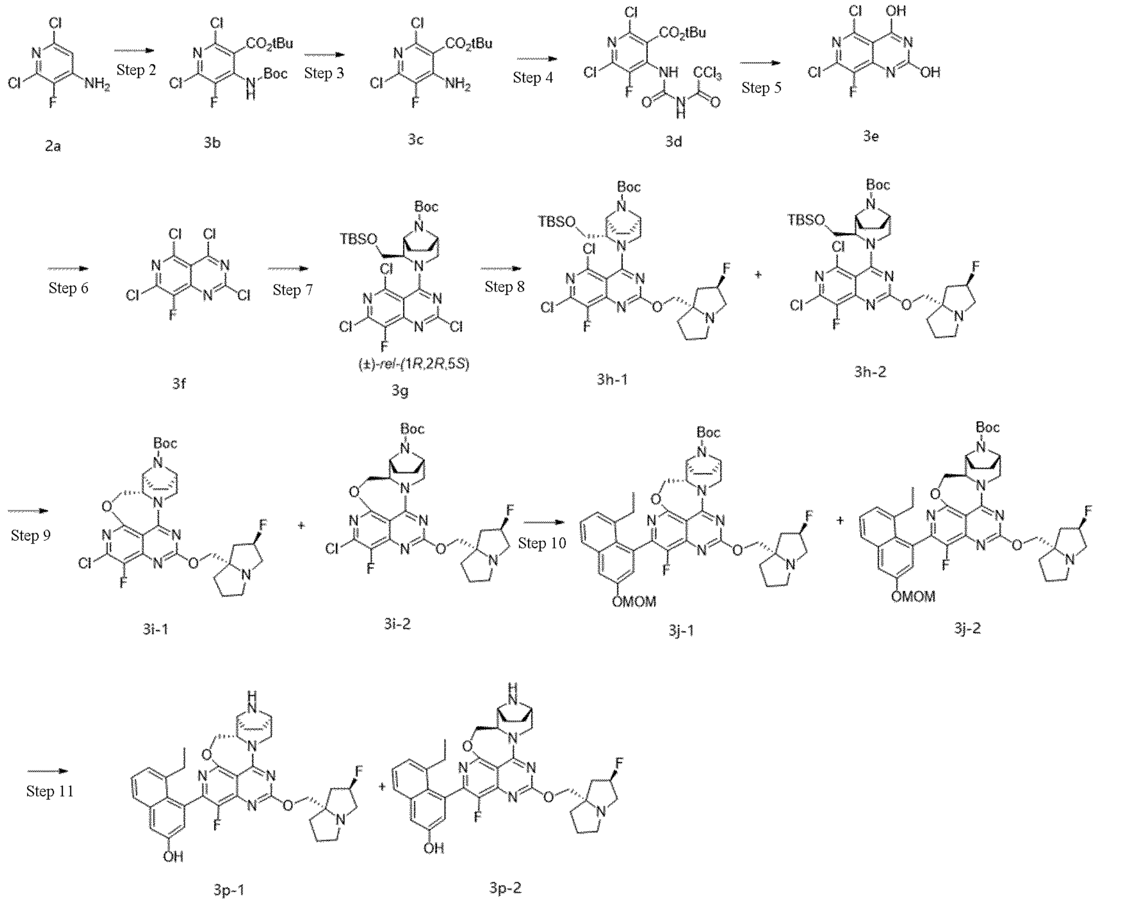


2a 3b 3c 3d 3e

3f (±)-*rel*-(1*R*,2*R*,5*S*) 3g 3h-1 + 3h-2

3i-1 + 3i-2 3j-1 + 3j-2

3p-1 + 3p-2

Step 1

*tert*-Butyl (±)-*rel*-(1*R*,2*R*,5*S*)-2-(((*tert*-butyldimethylsilyl)oxy)methyl)-3,8-diazabicyclo[3.2.1]octane-8-carboxylate **3a**

Compound **1g** (8.8 g, 36.3 mmol), *tert*-butyldimethylsilyl chloride (16 g, 106.1558 mmol), and 4-dimethylaminopyridine (4 g, 32.4739 mmol) were dissolved in 200 mL of dichloromethane, and triethylamine (15 g, 148.23 mmol, 21.4286 mL) was added. The mixture was stirred for 16 h and concentrated under reduced pressure, and the residue was purified by silica gel column chromatography with eluent system B to give title compound **3a** (8 g, yield: 61.7%).

MS m/z (ESI): 357.1[M+1].

Step 2

*tert*-Butyl 4-((*tert*-butoxycarbonyl)amino)-2,6-dichloro-5-fluoronicotinate **3b**

Compound **2a** (1.8 g, 9.94 mmol) was dissolved in tetrahydrofuran (50 mL), and 20 mL of a 2 M solution of sodium bis(trimethylsilyl)amide in tetrahydrofuran was added under an ice bath. The mixture was stirred for 0.5 h, and di-*tert*-butyl dicarbonate (6.5 g, 29.7 mmol) was then added. The mixture was stirred for 14 h, quenched with a saturated aqueous ammonium chloride solution, and extracted with ethyl acetate (50 mL × 3). The organic phases were combined, dried over anhydrous sodium sulfate, and filtered to remove the drying agent. Then the filtrate was concentrated under reduced pressure, and the residue was purified with eluent system B to give title compound **3b** (1 g, yield: 26.3%), which was directly used in the next step without purification.

MS m/z (ESI): 381.1[M+1].

Step 3

*tert*-Butyl 4-amino-2,6-dichloro-5-fluoronicotinate **3c**

Compound **3b** (1 g, 2.62 mmol) was dissolved in ethyl acetate (8 mL), and 3 mL of a 4 M solution of hydrochloric

acid in dioxane was added. The mixture was stirred for 2 h, made neutral with a 4 M aqueous sodium hydroxide solution under an ice bath, and extracted with ethyl acetate (10 mL × 3). The organic phases were combined, dried over anhydrous sodium sulfate, and filtered to remove the drying agent. The filtrate was concentrated under reduced pressure, and the residue was purified with eluent system B to give crude title compound **3c** (500 mg, yield: 67.8%).

MS m/z (ESI): 281.1[M+1].

Step 4

*tert*-Butyl 2,6-dichloro-5-fluoro-4-(3-(2,2,2-trichloroacetyl)ureido)nicotinate **3d**

Crude compound **3c** (500 mg, 1.77 mmol) was dissolved in tetrahydrofuran (10 mL), and trichloroacetyl isocyanate (670 mg, 3.55 mmol) was added. The mixture was stirred for 30 min and concentrated under reduced pressure to give crude title compound **3d** (835 mg, yield: 99.7%), which was directly used in the next step without purification.

MS m/z (ESI): 467.9[M+1].

Step 5

5,7-Dichloro-8-fluoro-pyrido[4,3-*d*]pyrimidine-2,4-diol **3e**

Crude compound **3d** (835 mg, 1.77 mmol) was dissolved in a 7 M methanolic ammonia solution (10 mL). The mixture was stirred for 1 h and concentrated under reduced pressure, and methyl *tert*-butyl ether (10 mL) was added to the residue. The mixture was stirred for 0.5 h and then filtered, and the filter cake was dried to give crude title compound **3e** (400 mg, yield: 89.9%), which was directly used in the next step without purification.

MS m/z (ESI): 249.9[M+1].

Step 6

2,4,5,7-Tetrachloro-8-fluoro-pyrido[4,3-d]pyrimidine **3f**

Crude compound **3e** (300 mg, 1.19 mmol) was dissolved in phosphorus oxychloride (6 mL), and *N,N*-diisopropylethylamine (800 mg, 6.19 mmol) was added. The mixture was stirred at 110 °C for 3 h, cooled to room temperature, and then concentrated under reduced pressure to give crude title compound **3f** (344 mg, yield: 97.7%), which was directly used in the next step without purification.

MS m/z (ESI): 285.8[M+1].

Step 7

*tert*-Butyl (±)-*rel*-(1*R*,2*R*,5*S*)-2-(((*tert*-butyldimethylsilyl)oxy)methyl)-3-(2,5,7-trichloro-8-fluoropyrido[4,3-*d*]pyrimidin-4-yl)-3,8-diazabicyclo[3.2.1]octane-8-carboxylate **3g**

Compound **3f** (1.0 g, 3.48 mmol) and *N,N*-diisopropylethylamine (0.9 g, 6.9 mmol) were dissolved in 15 mL of dichloromethane, and **3a** (1.25 g, 3.5 mmol) was added at -78 °C. The mixture was stirred at that temperature for 1 h, then reacted at room temperature for 16 h, and concentrated under reduced pressure, and the residue was purified with eluent system B to give crude title compound **3g** (1.56 g, yield: 73.7%).

MS m/z (ESI): 606.2[M+1].

Step 8

Diastereomeric mixture of *tert*-butyl (1*S*,2*S*,5*R*)-2-(((*tert*-butyldimethylsilyl)oxy)methyl)-3-(5,7-dichloro-8-fluoro-2-(((2*R*,7a*S*)-2-fluorotetrahydro-1*H*-pyrrolizin-7*a*(5*H*)-yl)methoxy)pyrido[4,3-*d*]pyrimidin-4-yl)-3,8-diazabicyclo[3.2.1]octane-8-carboxylate **3h-1** and

*tert*-butyl (1*R*,2*R*,5*S*)-2-(((tert-butyldimethylsilyl)oxy)methyl)-3-(5,7-dichloro-8-fluoro-2-(((2*R*,7*aS*)-2-fluorotetrahydro-1*H*-pyrrolizin-7*a*(5*H*)-yl)methoxy)pyrido[4,3-*d*]pyrimidin-4-yl)-3,8-diazabicyclo[3.2.1]octane-8-carboxylate **3h-2**

Compound **3g** (1.4 g, 2.3 mmol) was dissolved in 1,4-dioxane (20 mL), and ((2*R*,7*aS*)-2-fluorotetrahydro-1*H*-pyrrolizin-7*a*(5*H*)-yl)methanol (650 mg, 4.08 mmol, WuXi AppTec), *N,N*-diisopropylethylamine (1.5 g, 11.6 mmol), and a

4A molecular sieve (1.4 g) were added. The mixture was stirred at 105 °C for 6 h, cooled to room temperature, then filtered, and concentrated under reduced pressure to give a diastereomeric mixture of crude title compounds **3h-1 and 3h-2** (1.68 g, yield: 99.8%), which was directly used in the next step without purification.

MS m/z (ESI): 729.2[M+1]

Step 9

Diastereomeric mixture of *tert*-butyl (5*aS*,6*S*,9*R*)-2-chloro-1-fluoro-12-(((2*R*,7*aS*)-2-fluorotetrahydro-1*H*-pyrrolizin-7*a*(5iH)-yl)methoxy)-5*a*,6,7,8,9,10-hexahydro-5*H*-4-oxa-3,10*a*,11,13,14-pentaaza-6,9-methanonaphtho[1,8-*ab*]heptalen-14-carboxylate **3i-1** and

*tert*-butyl (5a*R*,6*R*,9*S*)-2-chloro-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-14-carboxylate **3i-2**

The diastereomeric mixture of crude compounds **3h-1** and **3h-2** (1.68 g, 2.3 mmol) was added into tetrabutylammonium fluoride (2.59 g, 11.51 mmol). The mixture was stirred at room temperature for 16 h and concentrated under reduced pressure, and the residue was purified with eluent system B to give a diastereomeric mixture of title compounds **3i-1 and 3i-2** (1.0 g, yield: 75.0%).

MS m/z (ESI): 579.2[M+1].

Step 10

Diastereomeric (1:1) mixture of *tert*-butyl (5*aS*,6*S*,9*R*)-2-(8-ethyl-3-(methoxymethyloxy)naphthalen-1-yl)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-14-carboxylate **3j-1** and

*tert*-butyl (5*aR*,6*R*,9*S*)-2-(8-ethyl-3-(methoxymethoxy)naphthalen-1-yl)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-14-carboxylate **3j-2**

The diastereomeric mixture of compounds **3i-1 and 3i-2** (1.9 g, 3.28 mmol), 2-(8-ethyl-3-(methoxymethoxy)naphthalen-1-yl)-4,4,5,5-tetramethyl-1,3,2-dioxaborolane (1.5 g, 4.38 mmol, prepared using the method for intermediate 21 disclosed on page 111 of the specification in Patent Application "WO2021/041671"), tetrakis(triphenylphosphine)palladium (1.16 g, 1 mmol, adamas), and cesium carbonate (4.7 g, 14.4 mmol) were dissolved in 36 mL of a mixed solution of 1,4-dioxane and water (V:V = 5:1). The mixture was reacted at 100 °C for 6 h in a nitrogen atmosphere and concentrated under reduced pressure to give a diastereomeric (1:1) mixture of crude title compounds **3j-1 and 3j-2** (2.5 g, yield: 100%).

MS m/z (ESI): 759.2[M+1].

Step 11

5-Ethyl-4-((5*aS*,6*S*,9*R*)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **3p-1**

5-Ethyl-4-((5*aR*,6*R*,9*S*)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **3p-2**

The diastereomeric mixture of crude compounds **3j-1** and **3j-2** (2.4 g, 3.16 mol) was dissolved in ethyl acetate (40 mL), and 17 mL of a 4 M solution of hydrochloric acid in dioxane was added. The mixture was reacted at 0 °C for 2 h and concentrated under reduced pressure, and the residue was purified by high performance liquid chromatography (Waters-2545, chromatography column: SharpSil-T C18, 30 × 150 mm, 5 μm; mobile phase: aqueous phase (10 mmol/L ammonium bicarbonate) and acetonitrile, gradient ratio: acetonitrile 38%-45%, flow rate: 30 mL/min) to give a diastereomeric (1:1) mixture of title compounds **3p-1 and 3p-2** (620 mg, yield: 32.6%).

MS m/z (ESI): 615.2[M+1].

$^{1}$H NMR (500 MHz, $CD_3OD$): δ 7.63 (d, 1H), 7.37 (q, 1H), 7.29 (q, 1H), 7.18 (dd, 1H), 7.08 (t, 1H), 5.48-5.29 (m, 2H), 4.55-4.28 (m, 4H), 4.17 (dd, 1H), 3.84-3.75 (m, 1H), 3.70 (t, 1H), 3.47 (q, 1H), 3.31-3.07 (m, 3H), 2.57-2.17 (m, 5H), 2.15-1.79 (m, 7H), 1.63 (t, 1H), 1.08-0.87 (m, 3H).

The diastereomeric mixture of compounds 3p-1 and **3p-2** was subjected to chiral resolution (Shimadzu LC-20AP,

chromatography column: DAICEL CHIRALPAK®IC, 25 × 250 mm, 10 μm; mobile phase A: n-hexane, mobile phase B: ethanol (0.1% 7 M NH3 in MeOH)), gradient ratio: A:B: 30:70, flow rate: 30 mL/min) to give title compounds **3p-1** (50 mg, yield: 35.7%) and **3p-2** (50 mg, yield: 35.7%).

Single-configuration compound (shorter retention time) **3p-2:** (50 mg, yield: 35.7%).

MS m/z (ESI): 615.2[M+1].

Chiral HPLC analysis: retention time: 9.85 min; purity: 99% (chromatography column: DAICEL CHIRALPAK®IC, 250 × 4.6 mm, 5 μm; mobile phase: n-hexane and ethanol (containing 0.2% diethylamine), flow rate: 1.0 mL/min).

$^1$H NMR (500 MHz, $CD_3OD$): δ 7.61-7.59 (m, 1H), 7.36-7.32 (m, 1H), 7.26-7.25 (m, 1H), 7.18-7.13 (m 1H), 7.05-6.95 (m, 1H), 5.37-5.22 (m, 2H), 5.09-5.00 (m, 1H), 4.61-4.56 (m, 1H), 4.49-4.41 (m, 1H), 4.30 (dd, 1H), 4.24-4.18 (m, 1H), 4.16-4.09 (m, 1H), 3.72 (dd, 1H), 3.62 (dd, 1H), 3.27-3.17 (m, 3H), 3.01 (td, 1H), 2.48 (dt, 1H), 2.40 - 2.10 (m, 5H), 1.99 (td, 2H), 1.94-1.75 (m, 4H), 0.99-0.88 m, 3H).

Single-configuration compound (longer retention time) **3p-1:** (50 mg, yield: 35.7%).

MS m/z (ESI): 615.2[M+1].

Chiral HPLC analysis: retention time: 16.0 min; purity: 99% (chromatography column: DAICEL CHIRALPAK®IC, 250 × 4.6 mm, 5 μm; mobile phase: n-hexane and ethanol (containing 0.2% diethylamine), flow rate: 1.0 mL/min).

$^1$H NMR (500 MHz, $CD_3OD$): δ 7.61-7.59 (m, 1H), 7.36-7.32 (m, 1H), 7.26-7.25 (m, 1H), 7.18-7.13 (m 1H), 7.05-6.94 (m, 1H), 5.36-5.33 (m, 2H), 5.10-5.01 (m, 1H), 4.61-4.56 (m, 1H), 4.49-4.41 (m, 1H), 4.30 (dd, 1H), 4.24-4.22 (m, 1H), 4.16-4.10 (m, 1H), 3.73-3.72 (m, 1H), 3.64-3.61 (m, 1H), 3.26-3.20 (m, 3H), 3.04-2.99 (m, 1H), 2.48 (dt, 1H), 2.38-2.17 (m, 5H), 2.03-1.96 (m, 2H), 1.93-1.78 (m, 4H), 0.99-0.88 m, 3H).

**Example 4**

5-Ethyl-6-fluoro-4-((5*aS*,6*S*,9*R*)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **4p-1**

5-Ethyl-6-fluoro-4-((5*aR*,6*R*,9*S*)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **4p-2**

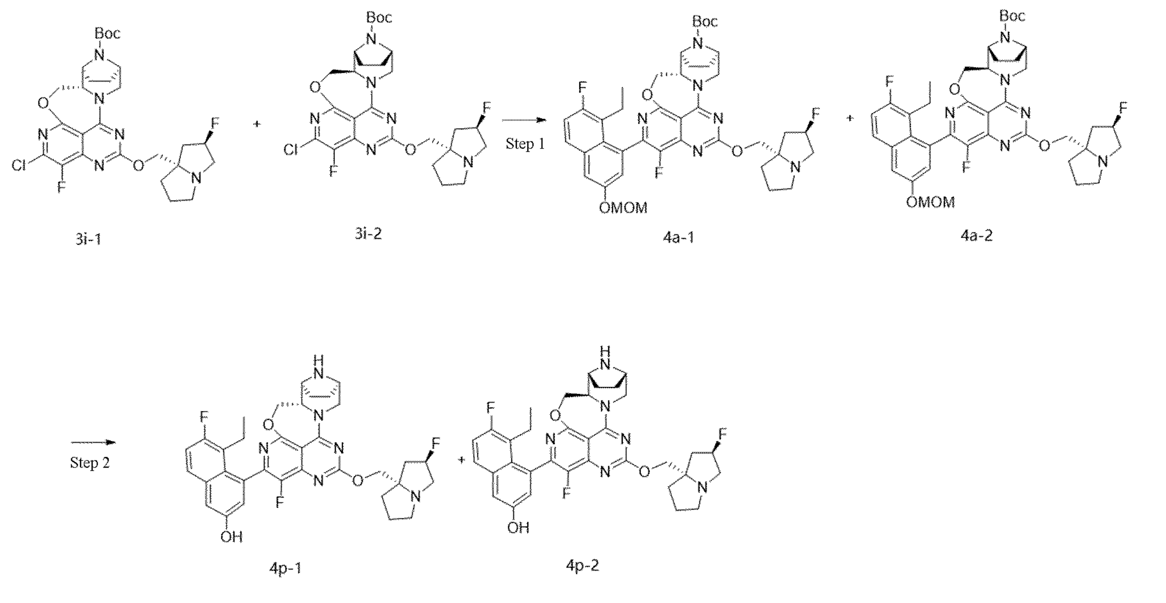


3i-1 3i-2 4a-1 4a-2

4p-1 4p-2

Step 10

Diastereomeric (1:1) mixture of *tert*-butyl (5*aS*,6*S*,9*R*)-2-(8-ethyl-7-fluoro-3-(methoxymethoxy)naphthalen-1-yl)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-14-carboxylate **4a-1** and

*tert*-butyl (5*aR*,6*R*,9*S*)-2-(8-ethyl-7-fluoro-3-(methoxymethoxy)naphthalen-1-yl)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-14-carboxylate **4a-2**

The diastereomeric mixture of compounds **3i-1 and 3i-2** (300 mg, 518.1 μmol), 2-(8-ethyl-7-fluoro-3-(methoxymethoxy)naphthalen-1-yl)-4,4,5,5-tetramethyl-1,3,2-dioxaborolane (280 mg, 777.2 μmol, prepared using the method for intermediate 18 disclosed on page 104 of the specification in Patent Application "WO2021/041671"), tetrakis(triphenylphosphine)palladium (120 mg, 103.8 μmol), and cesium carbonate (506 mg, 1.55 mmol) were dissolved in 6 mL of a mixed solution of 1,4-dioxane and water (V:V = 5:1). The mixture was reacted at 100 °C for 6 h in a nitrogen atmosphere and concentrated under reduced pressure to give a diastereomeric (1:1) mixture of title compounds **4a-1** and **4a-2** (400 mg), which was directly used in the next step without purification.
MS m/z (ESI): 777.2[M+1].

Step 11

5-Ethyl-6-fluoro-4-((5*aS*,6*S*,9*R*)-1-fluoro-12-(((2*R*,7*aS*)-2-fluorotetrahydro-1*H*-pyrrolizin-7a(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **4p-1**

5-Ethyl-6-fluoro-4-((5*aR*,6*R*,9*S*)-1-fluoro-12-(((2*R*,7*aS*)-2-fluorotetrahydro-1*H*-pyrrolizin-7*a*(5*H*)-yl)methoxy)-5*a*,6,7,8,9,10-hexahydro-5*H*-4-oxa-3,10*a*,11,13,14-pentaaza-6,9-methanonaphtho[1,8-*ab*]heptalen-2-yl)naphthalen-2-ol **4p-2**

The diastereomeric mixture of crude compounds **4a-1** and **4a-2** (160 mg, 205.9 μmol) was dissolved in ethyl acetate (5 mL), and 1 mL of a 4 M solution of hydrochloric acid in dioxane was added. The mixture was reacted at 0 °C for 2 h and concentrated under reduced pressure, and the residue was purified by high performance liquid chromatography (Waters-2545, chromatography column: SharpSil-T C18, 30 × 150 mm, 5 μm; mobile phase: aqueous phase (10 mmol/L ammonium bicarbonate) and acetonitrile, gradient ratio: acetonitrile 38%-45%, flow rate: 30 mL/min) to give a diastereomeric (1:1) mixture of title compounds **4p-1** (i.e., the compound represented by formula I) and **4p-2** (10 mg, yield: 7.2%).
MS m/z (ESI): 633.2[M+1].
$^1$H NMR (500 MHz, $CD_3OD$): δ 7.67 (ddd, 1H), 7.32-7.21 (m, 2H), 7.11-7.01 (m, 1H), 5.38-5.35 (m, 2H), 5.11-5.03 (m, 1H), 4.64-4.59 (m, 1H), 4.52-4.46 (m, 1H), 4.34-4.29 (m, 1H), 4.25 (dd, 1H), 4.18-4.12 (m, 1H), 3.74 (br, 1H), 3.65 (br, 1H), 3.26-3.23 (m, 3H), 3.05-3.01 (m, 1H), 2.61-1.81 (m, 12H), 0.94-0.82 (m, 3H).
The diastereomeric mixture of compounds **4p-1** and **4p-2** was subjected to chiral resolution (Shimadzu LC-20AP, chromatography column: DAICEL CHIRALPAK®IC, 25 × 250 mm, 10 μm; mobile phase A: n-hexane, mobile phase B: ethanol (0.1% 7 M NH3 in MeOH)), gradient ratio: A:B: 40:60, flow rate: 30 mL/min) to give title compounds **4p-1** (26 mg, yield: 43.3%) and **4p-2** (26 mg, yield: 43.3%).
Single-configuration compound (shorter retention time) **4p-2:** (26 mg, yield: 43.3%).
MS m/z (ESI): 633.2[M+1].
Chiral HPLC analysis: retention time: 7.89 min; purity: 99% (chromatography column: DAICEL CHIRALPAK®IC, 250 × 4.6 mm, 5 μm; mobile phase: n-hexane and ethanol (containing 0.2% diethylamine), flow rate: 1.0 mL/min).
$^1$H NMR (500 MHz, $CD_3OD$): δ 7.67 (ddd, 1H), 7.32-7.21 (m, 2H), 7.11-7.01 (m, 1H), 5.38-5.27 (m, 2H), 5.11-5.03 (m, 1H), 4.64-4.59 (m, 1H), 4.52-4.44 (m, 1H), 4.33 (d, 1H), 4.24 (dd, 1H), 4.18-4.12 (m, 1H), 3.75 (br, 1H), 3.66 (br, 1H), 3.27-3.18 (m, 3H), 3.05-3.03 (m, 1H), 2.60-1.81 (m, 12H), 0.93-0.82 (m, 3H).
Single-configuration compound (longer retention time) **4p-1:** (26 mg, yield: 43.3%).
MS m/z (ESI): 633.2[M+1].
Chiral HPLC analysis: retention time: 13.8 min; purity: 99% (chromatography column: DAICEL CHIRALPAK®IC, 250 × 4.6 mm, 5 μm; mobile phase: n-hexane and ethanol (containing 0.2% diethylamine), flow rate: 1.0 mL/min).
$^1$H NMR (500 MHz, $CD_3OD$): δ 7.67 (ddd, 1H), 7.32-7.21 (m, 2H), 7.11-7.01 (m, 1H), 5.38-5.35 (m, 2H), 5.11-5.03 (m, 1H), 4.64-4.59 (m, 1H), 4.52-4.46 (m, 1H), 4.34-4.29 (m, 1H), 4.25 (dd, 1H), 4.18-4.12 (m, 1H), 3.74 (br, 1H), 3.65 (br, 1H), 3.26-3.23 (m, 3H), 3.05-3.01 (m, 1H), 2.61-1.81 (m, 12H), 0.94-0.82 m, 3H).

**Example 5**

| | | Amount of addition |
|---|---|---|
| Lipid solution | Hydrogenated soy phosphatidylcholine | 1215.0 mg |
| | Cholesterol | 398.1 mg |
| | Phosphatidylethanolamine pegol (mPEG2000-DSPE) | 160.2 mg |
| | Absolute ethanol | 6 mL |
| Compound solution | Compound of formula I | 45 mg |
| | Absolute ethanol | q.s. |
| | Hydrochloric acid | q.s. |
| Aqueous phase solution 1 | Copper gluconate | 8169.12 mg |
| | Triethanolamine | q.s. |
| | Water | 180 mL |
| Aqueous phase solution 2 | Histidine hydrochloride | 3720.0 mg |
| | Sodium chloride | 21.6 g |
| | Sodium hydroxide | q.s. |
| | Hydrochloric acid | q.s. |
| | Water | 2400 mL |

Copper gluconate was weighed out and uniformly dissolved in a proper amount of water by stirring. The pH value of the solution was adjusted to 7.4 with triethanolamine, and the solution was brought to volume (180 mL) to give an aqueous phase solution 1. Histidine hydrochloride and sodium chloride were weighed out and dissolved in a proper amount of water. The pH value of the solution was adjusted to 7.4 with sodium hydroxide and/or hydrochloric acid, and the solution was brought to volume (2400 mL) to give an aqueous phase solution 2.

Hydrogenated soy phosphatidylcholine, cholesterol, and phosphatidylethanolamine pegol were weighed out, added to absolute ethanol, and dissolved by heating and stirring to give a lipid solution. The lipid solution was added to the aqueous phase solution 1 described above. The resulting mixture was stirred for about 15 min at a temperature higher than the phospholipid phase transition temperature. Then hydration was further performed, and most of the ethanol was removed under reduced pressure. The residue was extruded by an extruder and sized to give a blank liposome. The blank liposome was treated by a tangential flow filtration system, and the outer aqueous phase of the liposome was replaced with the aqueous phase solution 2. The compound of formula I was dissolved in an ethanol solution, and a proper amount of hydrochloric acid was added to give a 120 mg/mL compound solution. The compound solution was slowly added to the blank liposome. The mixture was incubated at a temperature higher than the phospholipid phase transition temperature for 15 min and then rapidly cooled to room temperature. The product was concentrated to the target concentration using a tangential flow system, and the ethanol residue was further removed. The concentrate was brought to volume to give a liposome product. The product was characterized, with information shown in the table below.

| Product | Content (mg/mL) | Encapsulation efficiency | Particle size of product (nm) | | | |
|---|---|---|---|---|---|---|
| | | | Average particle size (calculated in terms of light intensity) | D (10) | D (50) | D (90) |
| Liposome of Example 5 | 0.73 | 99.9% | 75 | 55 | 77 | 107 |

**Example 6**

| | | Amount of addition |
|---|---|---|
| Lipid solution | Hydrogenated soy phosphatidylcholine | 1215.0 mg |
| | Cholesterol | 398.1 mg |
| | Phosphatidylethanolamine pegol | 120 mg |
| | Absolute ethanol | 6 mL |
| Compound solution | Compound of formula I | 180 mg |
| | Absolute ethanol | q.s. |
| | Hydrochloric acid | q.s. |
| Aqueous phase solution 1 | Ammonium sulfate | 7135.6 mg |
| | Phosphoric acid | 1245.2 mg |
| | Ammonia water | q.s. |
| | Purified water | 180 mL |
| Aqueous phase solution 2 | Histidine hydrochloride | 3720.0 mg |
| | Sodium chloride | 21.6 g |
| | Sodium hydroxide | q.s. |
| | Dilute hydrochloric acid | q.s. |
| | Purified water | 2400 mL |

Ammonium sulfate and phosphoric acid were weighed out and uniformly dissolved in a proper amount of water by stirring. The pH value of the solution was adjusted to 5.85 with ammonia water, and the solution was brought to volume (180 mL) to give an aqueous phase solution 1. Histidine hydrochloride and sodium chloride were weighed out and dissolved in a proper amount of water. The pH value of the solution was adjusted to 6.35 with sodium hydroxide and/or hydrochloric acid, and the solution was brought to volume (2400 mL) to give an aqueous phase solution 2.

Hydrogenated soy phosphatidylcholine, cholesterol, and phosphatidylethanolamine pegol were weighed out, added to absolute ethanol, and dissolved by heating and stirring to give a lipid solution. The lipid solution was added to the aqueous phase solution 1 described above. The resulting mixture was stirred for about 15 min at a temperature higher than the phospholipid phase transition temperature. Then hydration was further performed, and most of the ethanol was removed under reduced pressure. The residue was extruded by an extruder and sized to give a blank liposome. The blank liposome was treated by a tangential flow filtration system, and the outer aqueous phase of the liposome was replaced with the aqueous phase solution 2. The compound of formula I was dissolved in an ethanol solution, and a proper amount of hydrochloric acid was added to give a 120 mg/mL compound solution. The compound solution was slowly added to the blank liposome. The mixture was incubated at a temperature higher than the phospholipid phase transition temperature for 15 min and then rapidly cooled to room temperature. The product was concentrated to the target concentration using a tangential flow system, and the ethanol residue was further removed. The concentrate was brought to volume to give a liposome product. The product was characterized, with information shown in the table below.

| Product | Content (mg/mL) | Encapsulation efficiency | Particle size of product (nm) | | | |
|---|---|---|---|---|---|---|
| | | | Average particle size (calculated in terms of light intensity) | D (10) | D (50) | D (90) |
| Liposome of Example 6 | 3.14 | 99.5% | 73 | 54 | 75 | 103 |

**Example 7**

| | | Amount of addition |
|---|---|---|
| Lipid solution | Hydrogenated soy phosphatidylcholine | 1215.0 mg |
| | Cholesterol | 398.1 mg |
| | Phosphatidylethanolamine pegol | 120 mg |
| | Absolute ethanol | 6 mL |
| Compound solution | Compound of formula I | 180 mg |
| | Absolute ethanol | q.s. |
| | Hydrochloric acid | q.s. |
| Aqueous phase solution 1 | Ammonium sulfate | 7135.6 mg |
| | Phosphoric acid | 1245.2 mg |
| | Ammonia water | q.s. |
| | Purified water | 180 mL |
| Aqueous phase solution 2 | Histidine hydrochloride | 3720.0 mg |
| | Sodium chloride | 21.6 g |
| | Sodium hydroxide | q.s. |
| | Dilute hydrochloric acid | q.s. |
| | Purified water | 2400 mL |

Ammonium sulfate and phosphoric acid were weighed out and uniformly dissolved in a proper amount of water by stirring. The pH value of the solution was adjusted to 7.0 with ammonia water, and the solution was brought to volume (180 mL) to give an aqueous phase solution 1. Histidine hydrochloride and sodium chloride were weighed out and dissolved in a proper amount of water. The pH value of the solution was adjusted to 7.5 with sodium hydroxide and/or hydrochloric acid, and the solution was brought to volume (2400 mL) to give an aqueous phase solution 2.

Hydrogenated soy phosphatidylcholine, cholesterol, and phosphatidylethanolamine pegol were weighed out, added to absolute ethanol, and dissolved by heating and stirring to give a lipid solution. The lipid solution was added to the aqueous phase solution 1 described above. The resulting mixture was stirred for about 15 min at a temperature higher than the phospholipid phase transition temperature. Then hydration was further performed, and most of the ethanol was removed under reduced pressure. The residue was extruded by an extruder and sized to give a blank liposome. The blank liposome was treated by a tangential flow filtration system, and the outer aqueous phase of the liposome was replaced with the aqueous phase solution 2. The compound of formula I was dissolved in an ethanol solution, and a proper amount of hydrochloric acid was added to give a 120 mg/mL compound solution. The compound solution was slowly added to the blank liposome. The mixture was incubated at a temperature higher than the phospholipid phase transition temperature for 15 min and then rapidly cooled to room temperature. The product was concentrated to the target concentration using a tangential flow system, and the ethanol residue was further removed. The concentrate was brought to volume to give a liposome product. The product was characterized, with information shown in the table below.

| Product | Drug loading encapsulation efficiency (%) |
|---|---|
| Liposome of Example 7 | 99.7 |

**Example 8**

| | | Amount of addition |
|---|---|---|
| Lipid solution | Hydrogenated soy phosphatidylcholine | 1215.0 mg |
| | Cholesterol | 398.1 mg |
| | Phosphatidylethanolamine pegol | 120 mg |
| | Absolute ethanol | 6 mL |
| Compound solution | Compound of formula I | 180 mg |
| | Absolute ethanol | q.s. |
| | Hydrochloric acid | q.s. |
| Aqueous phase solution 1 | Ammonium sulfate | 7135.6 mg |
| | Citric acid monohydrate | 2268.1 mg |
| | Ammonia water | q.s. |
| | Purified water | 180 mL |
| Aqueous phase solution 2 | Histidine hydrochloride | 3720.0 mg |
| | Sodium chloride | 21.6 g |
| | Sodium hydroxide | q.s. |
| | Dilute hydrochloric acid | q.s. |
| | Purified water | 2400 mL |

Ammonium sulfate and citric acid monohydrate were weighed out and uniformly dissolved in a proper amount of water by stirring. The pH value of the solution was adjusted to 7.0 with ammonia water, and the solution was brought to volume (180 mL) to give an aqueous phase solution 1. Histidine hydrochloride and sodium chloride were weighed out and dissolved in a proper amount of water. The pH value of the solution was adjusted to 7.5 with sodium hydroxide and/or hydrochloric acid, and the solution was brought to volume (2400 mL) to give an aqueous phase solution 2.

Hydrogenated soy phosphatidylcholine, cholesterol, and phosphatidylethanolamine pegol were weighed out, added to absolute ethanol, and dissolved by heating and stirring to give a lipid solution. The lipid solution was added to the aqueous phase solution 1 described above. The resulting mixture was stirred for about 15 min at a temperature higher than the phospholipid phase transition temperature. Then hydration was further performed, and most of the ethanol was removed under reduced pressure. The residue was extruded by an extruder and sized to give a blank liposome. The blank liposome was treated by a tangential flow filtration system, and the outer aqueous phase of the liposome was replaced with the aqueous phase solution 2. The compound of formula I was dissolved in an ethanol solution, and a proper amount of hydrochloric acid was added to give a 120 mg/mL compound solution. The compound solution was slowly added to the blank liposome. The mixture was incubated at a temperature higher than the phospholipid phase transition temperature for 15 min and then rapidly cooled to room temperature. The product was concentrated to the target concentration using a tangential flow system, and the ethanol residue was further removed. The concentrate was brought to volume to give a liposome product. The product was characterized, with information shown in the table below.

| Product | Drug loading encapsulation efficiency (%) |
|---|---|
| Liposome of Example 8 | 99.7 |

**Example 9**

| | | Amount of addition |
|---|---|---|
| Lipid solution | Hydrogenated soy phosphatidylcholine | 1215.0 mg |
| | Cholesterol | 398.1 mg |
| | Phosphatidylethanolamine pegol | 120 mg |
| | Absolute ethanol | 6 mL |
| Compound solution | Compound of formula I | 180 mg |
| | Absolute ethanol | q.s. |
| | Hydrochloric acid | q.s. |
| Aqueous phase solution 1 (pH = 7.0) | Ammonium sulfate | 7135.6 mg |
| | Malic acid | 1447.2 mg |
| | Ammonia water | q.s. |
| | Purified water | 180 mL |
| Aqueous phase solution 2 (pH = 7.5) | Histidine hydrochloride | 3720.0 mg |
| | Sodium chloride | 21.6 g |
| | Sodium hydroxide | q.s. |
| | Dilute hydrochloric acid | q.s. |
| | Purified water | 2400 mL |

Ammonium sulfate and malic acid were weighed out and uniformly dissolved in a proper amount of water by stirring. The pH value of the solution was adjusted to 7.0 with ammonia water, and the solution was brought to volume (180 mL) to give an aqueous phase solution 1. Histidine hydrochloride and sodium chloride were weighed out and dissolved in a proper amount of water. The pH value of the solution was adjusted to 7.5 with sodium hydroxide and/or hydrochloric acid, and the solution was brought to volume (2400 mL) to give an aqueous phase solution 2.

Hydrogenated soy phosphatidylcholine, cholesterol, and phosphatidylethanolamine pegol were weighed out, added to absolute ethanol, and dissolved by heating and stirring to give a lipid solution. The lipid solution was added to the aqueous phase solution 1 described above. The resulting mixture was stirred for about 15 min at a temperature higher than the phospholipid phase transition temperature. Then hydration was further performed, and most of the ethanol was removed under reduced pressure. The residue was extruded by an extruder and sized to give a blank liposome. The blank liposome was treated by a tangential flow filtration system, and the outer aqueous phase of the liposome was replaced with the aqueous phase solution 2. The compound of formula I was dissolved in an ethanol solution, and a proper amount of hydrochloric acid was added to give a 120 mg/mL compound solution. The compound solution was slowly added to the blank liposome. The mixture was incubated at a temperature higher than the phospholipid phase transition temperature for 15 min and then rapidly cooled to room temperature. The product was concentrated to the target concentration using a tangential flow system, and the ethanol residue was further removed. The concentrate was brought to volume to give a liposome product. The product was characterized, with information shown in the table below.

| Product | Drug loading encapsulation efficiency (%) |
|---|---|
| Liposome of Example 9 | 99.7 |

**Example 10**

| | | Amount of addition |
|---|---|---|
| Lipid solution | Hydrogenated soy phosphatidylcholine | 1215.0 mg |
| | Cholesterol | 398.1 mg |
| | Phosphatidylethanolamine pegol | 120 mg |
| | Absolute ethanol | 6 mL |
| Compound solution | Compound of formula I | 180 mg |
| | Absolute ethanol | q.s. |
| | Hydrochloric acid | q.s. |
| Aqueous phase solution 1 (pH = 7.0) | Ammonium sulfate | 7135.6 mg |
| | Histidine hydrochloride | 2264 mg |
| | Ammonia water | q.s. |
| | Purified water | 180 mL |
| Aqueous phase solution 2 (pH = 7.5) | Histidine hydrochloride | 3720.0 mg |
| | Sodium chloride | 21.6 g |
| | Sodium hydroxide | q.s. |
| | Dilute hydrochloric acid | q.s. |
| | Purified water | 2400 mL |

Ammonium sulfate and histidine hydrochloride were weighed out and uniformly dissolved in a proper amount of water by stirring. The pH value of the solution was adjusted to 7.0 with ammonia water, and the solution was brought to volume (180 mL) to give an aqueous phase solution 1. Histidine hydrochloride and sodium chloride were weighed out and dissolved in a proper amount of water. The pH value of the solution was adjusted to 7.5 with sodium hydroxide and/or hydrochloric acid, and the solution was brought to volume (2400 mL) to give an aqueous phase solution 2.

Hydrogenated soy phosphatidylcholine, cholesterol, and phosphatidylethanolamine pegol were weighed out, added to absolute ethanol, and dissolved by heating and stirring to give a lipid solution. The lipid solution was added to the aqueous phase solution 1 described above. The resulting mixture was stirred for about 15 min at a temperature higher than the phospholipid phase transition temperature. Then hydration was further performed, and most of the ethanol was removed under reduced pressure. The residue was extruded by an extruder and sized to give a blank liposome. The blank liposome was treated by a tangential flow filtration system, and the outer aqueous phase of the liposome was replaced with the aqueous phase solution 2. The compound of formula I was dissolved in an ethanol solution, and a proper amount of hydrochloric acid was added to give a 120 mg/mL compound solution. The compound solution was slowly added to the blank liposome. The mixture was incubated at a temperature higher than the phospholipid phase transition temperature for 15 min and then rapidly cooled to room temperature. The product was concentrated to the target concentration using a tangential flow system, and the ethanol residue was further removed. The concentrate was brought to volume to give a liposome product. The product was characterized, with information shown in the table below.

| Product | Drug loading encapsulation efficiency (%) |
|---|---|
| Liposome of Example 10 | 99.6 |

**Example 11**

| | | Amount of addition |
|---|---|---|
| Lipid solution | Hydrogenated soy phosphatidylcholine | 978 mg |
| | Cholesterol | 318 mg |
| | Phosphatidylethanolamine pegol | 120 mg |
| | Absolute ethanol | 6 mL |
| Compound solution | Compound of formula I | 180 mg |
| | Absolute ethanol | q.s. |
| | Hydrochloric acid | q.s. |
| Aqueous phase solution 1 (pH = 5.85) | Ammonium sulfate | 7135.6 mg |
| | Phosphoric acid | 1245.2 mg |
| | Ammonia water | q.s. |
| | Purified water | 180 mL |
| Aqueous phase solution 2 (pH = 6.35) | Histidine hydrochloride | 3720.0 mg |
| | Sodium chloride | 21.6 g |
| | Sodium hydroxide | q.s. |
| | Dilute hydrochloric acid | q.s. |
| | Purified water | 2400 mL |

Ammonium sulfate and phosphoric acid were weighed out and uniformly dissolved in a proper amount of water by stirring. The pH value of the solution was adjusted to 5.85 with ammonia water, and the solution was brought to volume (180 mL) to give an aqueous phase solution 1. Histidine hydrochloride and sodium chloride were weighed out and dissolved in a proper amount of water. The pH value of the solution was adjusted to 6.35 with sodium hydroxide and/or hydrochloric acid, and the solution was brought to volume (2400 mL) to give an aqueous phase solution 2.

Hydrogenated soy phosphatidylcholine, cholesterol, and phosphatidylethanolamine pegol were weighed out, added to absolute ethanol, and dissolved by heating and stirring to give a lipid solution. The lipid solution was added to the aqueous phase solution 1 described above. The resulting mixture was stirred for about 15 min at a temperature higher than the phospholipid phase transition temperature. Then hydration was further performed, and most of the ethanol was removed under reduced pressure. The residue was extruded by an extruder and sized to give a blank liposome. The blank liposome was treated by a tangential flow filtration system, and the outer aqueous phase of the liposome was replaced with the aqueous phase solution 2. The compound of formula I was dissolved in an ethanol solution, and a proper amount of hydrochloric acid was added to give a 120 mg/mL compound solution. The compound solution was slowly added to the blank liposome. The mixture was incubated at a temperature higher than the phospholipid phase transition temperature for 15 min and then rapidly cooled to room temperature. The product was concentrated to the target concentration using a tangential flow system, and the ethanol residue was further removed. The concentrate was brought to volume to give a liposome product. The product was characterized, with information shown in the table below.

| Product | Average particle size (nm) | Drug loading encapsulation efficiency (%) |
|---|---|---|
| Liposome of Example 11 | 85 | 99.0 |

**Example 12**

| | | Amount of addition |
|---|---|---|
| Lipid solution | Hydrogenated soy phosphatidylcholine | 1215 mg |
| | Cholesterol | 318 mg |
| | Phosphatidylethanolamine pegol | 120 mg |
| | Absolute ethanol | 6 mL |
| Compound solution | Compound of formula I | 180 mg |
| | Absolute ethanol | q.s. |
| | Hydrochloric acid | q.s. |
| Aqueous phase solution 1 (pH = 5.85) | Ammonium sulfate | 7135.6 mg |
| | Phosphoric acid | 1245.2 mg |
| | Ammonia water | q.s. |
| | Purified water | 180 mL |
| Aqueous phase solution 2 (pH = 6.35) | Histidine hydrochloride | 3720.0 mg |
| | Sodium chloride | 21.6 g |
| | Sodium hydroxide | q.s. |
| | Dilute hydrochloric acid | q.s. |
| | Purified water | 2400 mL |

Ammonium sulfate and phosphoric acid were weighed out and uniformly dissolved in a proper amount of water by stirring. The pH value of the solution was adjusted to 5.85 with ammonia water, and the solution was brought to volume (180 mL) to give an aqueous phase solution 1. Histidine hydrochloride and sodium chloride were weighed out and dissolved in a proper amount of water. The pH value of the solution was adjusted to 6.35 with sodium hydroxide and/or hydrochloric acid, and the solution was brought to volume (2400 mL) to give an aqueous phase solution 2.

Hydrogenated soy phosphatidylcholine, cholesterol, and phosphatidylethanolamine pegol were weighed out, added to absolute ethanol, and dissolved by heating and stirring to give a lipid solution. The lipid solution was added to the aqueous phase solution 1 described above. The resulting mixture was stirred for about 15 min at a temperature higher than the phospholipid phase transition temperature. Then hydration was further performed, and most of the ethanol was removed under reduced pressure. The residue was extruded by an extruder and sized to give a blank liposome. The blank liposome was treated by a tangential flow filtration system, and the outer aqueous phase of the liposome was replaced with the aqueous phase solution 2. The compound of formula I was dissolved in an ethanol solution, and a proper amount of hydrochloric acid was added to give a 120 mg/mL compound solution. The compound solution was slowly added to the blank liposome. The mixture was incubated at a temperature higher than the phospholipid phase transition temperature for 15 min and then rapidly cooled to room temperature. The product was concentrated to the target concentration using a tangential flow system, and the ethanol residue was further removed. The concentrate was brought to volume to give a liposome product. The product was characterized, with information shown in the table below.

| Product | Average particle size (nm) | Drug loading encapsulation efficiency (%) |
|---|---|---|
| Liposome of Example 12 | 81 | 97.4 |

## Biological Evaluations

### Test Example 1: Biological Evaluation of Inhibition of ERK Phosphorylation in AGS Cells (HTRF Method)

I. Objective

In the experiment, the inhibitory effect of the compound represented by formula I on the KRAS target was evaluated by assessing the inhibitory effect of the compound on ERK phosphorylation in cells and based on the $IC_{50}$.

II. Experimental method

AGS cells (Nanjing Cobioer, CBP60476) were cultured in an RPMI1640 (Hyclone, SH30809.01) complete culture medium containing 10% fetal bovine serum. On the first day of the experiment, the AGS cells were seeded into a 96-well plate with a complete culture medium at a density of 40,000 cells/well, with each well containing 190 μL of a cell suspension. The plate was incubated overnight in a cell incubator at 37 °C with 5% $CO_2$.

The next day, the test compound, serially diluted in a complete culture medium, was added at 10 μL/well. The final concentrations of the compound were 9 concentration points obtained by 5-fold serial dilution from 10 μM. A blank control containing 0.5% DMSO was set. The plate was incubated in a cell incubator at 37 °C with 5% $CO_2$ for 1 h. After the incubation, the 96-well cell culture plate was taken out, and the culture medium was removed using a pipette. The plate was washed once by adding 200 μL of PBS (Shanghai BasalMedia Technologies Co., Ltd., B320) to each well. The PBS was removed using a pipette, and 50 μL of a lysis buffer (Cisbio, 64KL1FDF) containing a blocking reagent (Cisbio, 64KB 1AAC) was added to each well. The plate was shaken on a shaker at room temperature for 40 min for lysis. After lysis, the lysate was pipetted for good uniformity. 16 μL of the lysate in each well was transferred to two HTRF 96-well assay plates (Cisbio, 66PL96100), and then 4 μL of a premixed phosphorylated ERK1/2 antibody solution (Cisbio, 64AERPEG) or 4 μL of a premixed total ERK1/2 antibody solution (Cisbio, 64NRKPEG) was added to these two plates. The plates were sealed with a plate sealer film, centrifuged for 1 min in a microplate centrifuge, and incubated overnight at room temperature in the dark.

On the third day, the fluorescence values at an excitation wavelength of 337 nm and emission wavelengths of 665 nm and 620 nm were obtained using an ENVISION multimode microplate reader (PerkinElmer, ENVISION).

III. Data analysis and results

The $IC_{50}$ value for the inhibitory activity of the compound was calculated using Graphpad Prism software based on the compound concentration and the phosphorylated ERK/total ERK ratio. The result is shown in Table 1 below.

Table 1. Inhibitory activity data for ERK phosphorylation in AGS cells

| Compound No. | AGS/$IC_{50}$ (nM) |
|---|---|
| Compound represented by formula I | 0.4 |
| Conclusion: The compound represented by formula I has a relatively good inhibitory effect on ERK phosphorylation in AGS cells. | |

**Test Example 2: Biological Evaluation of Inhibition of 3D Proliferation of GP2d and AGS Cells**

I. Objective

The inhibitory effect of the compound represented by formula I on the KRAS target was evaluated by assessing the inhibitory effect of the compound represented by formula I on the 3D proliferation of GP2d and AGS cells.

II. Experimental method

GP2d cells (Nanjing Cobioer, CBP60010) were cultured in a complete culture medium, namely a DMEM/high glucose culture medium (Hyclone, SH30243.01) containing 10% fetal bovine serum (Corning, 35-076-CV). On the first day of the experiment, the GP2d cells were seeded into a 96-well low-adsorption plate (Corning, CLS7007-24EA) with a complete culture medium at a density of 1000 cells/well, with each well containing 90 μL of a cell suspension. The plate was centrifuged at 2000 rpm for 5 min at room temperature and then incubated overnight in a cell incubator at 37 °C with 5% $CO_2$.

AGS cells (Nanjing Cobioer, CBP60476) were cultured in a complete culture medium, namely an RPMI1640 medium (Hyclone, SH30809.01) containing 10% fetal bovine serum (Corning, 35-076-CV). On the first day of the experiment, the AGS cells were seeded into a 96-well low-adsorption plate (Corning, CLS7007-24EA) with a complete culture medium at a density of 1000 cells/well, with each well containing 90 μL of a cell suspension. The plate was centrifuged at 2000 rpm for 5 min at room temperature and then incubated overnight in a cell incubator at 37 °C with 5% $CO_2$.

The next day, the test compound, serially diluted in a complete culture medium, was added at 10 μL/well. The final concentrations of the compound for GP2d cells were 9 concentration points obtained by 3-fold serial dilution from 1 μM, and the final concentrations of the compound for AGS cells were 9 concentration points obtained by 3-fold serial dilution

from 10 μM. A blank control containing 0.5% DMSO was set for both types of cells. The plates were incubated in a cell incubator at 37 °C with 5% $CO_2$ for 120 h. On the seventh day, the 96-well cell culture plates were taken out, and 50 μL of the CellTiter-Glo® 3D reagent (Promega, G9682) was added to each well. The plates were shaken at room temperature for 25 min. The contents in the wells were pipetted for good uniformity, and 50 μL of the mixture was transferred into a white impermeable 96-well plate (PE, 6005290). The luminescence signal values were obtained using a multimode microplate reader (PerkinElmer, ENVISION).

III. Data analysis and results

$IC_{50}$ values for the inhibitory activity of the compound were calculated using Graphpad Prism software, and the results are shown in Table 2 below.

Table 2. Inhibitory activity data for the 3D proliferation of AGS and GP2d cells

| Compound No. | AGS /$IC_{50}$ (nM) | GP2d /$IC_{50}$ (nM) |
|---|---|---|
| Compound represented by formula I | 5.8 | 0.9 |
| Conclusion: The compound represented by formula I has a relatively good inhibitory effect on the 3D proliferation of AGS and GP2d cells. | | |

**Test Example 3: Determination of Affinity of Compound for KRAS Protein Subtype G12D or WT by SPR Method**

Biotinylated Avi-KRAS-WT or Avi-KRAS-G12D was diluted to 20 μg/mL with a 1× HBS-P+ (Cat. # BR1006-71) buffer containing 100 mM $MgCl_2$ and then allowed to flow through channel 2 of an SA (Cat. # BR1005-31) biosensor chip for 420 s to obtain a coupling level of approximately 5000-7000 RU. Then samples of the compound were injected sequentially in descending order for 120 s, followed by 720 s of dissociation. The testing was performed using a single-cycle kinetic mode. Reaction signals were detected in real time using a Biacore 8K instrument to obtain association and dissociation curves. After the testing was finished, data analysis was performed using Biacore 8K evaluation software. Data fitting was performed using a 1:1 model, and affinity data were obtained. The results are shown in Table 3 below.

| Compound No. | G12D KD (mol/L) | WT KD (mol/L) |
|---|---|---|
| Compound represented by formula I | 0.03E-09 | 1.54E-09 |
| Conclusion: The compound represented by formula I has a relatively good affinity for KRAS protein subtype G12D or WT. | | |

## Claims

1. A pharmaceutical composition, comprising a compound of formula I or a pharmaceutically acceptable salt thereof and a lipid,

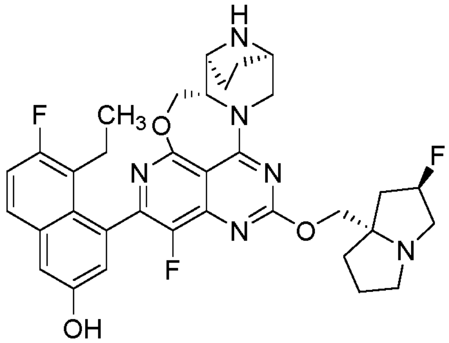

(I)

.

2. The pharmaceutical composition according to claim 1, wherein the pharmaceutical composition is a liposome.

3. The pharmaceutical composition according to claim 1 or 2, wherein the lipid comprises at least one phospholipid, and the phospholipid is preferably dipalmitoylphosphatidylcholine (DPPC), distearoylphosphatidylcholine (DSPC), dimyristoylphosphatidylcholine (DMPC), 1-palmitoyl-2-linoleoyl-sn-glycero-3-phosphatidylcholine (PLPC), dioleoylphosphatidylcholine (DOPC), dierucoylphosphatidylcholine (DEPC), egg yolk phosphatidylcholine (EPC), dilauroylphosphatidylcholine (DLPC), hydrogenated soy phosphatidylcholine (HSPC), 1-myristoyl-2-palmitoylphosphatidylcholine (MPPC), 1-palmitoyl-2-myristoylphosphatidylcholine (PMPC), 1-palmitoyl-2-stearoyl phosphatidylcholine (PSPC), 1-stearoyl-2-palmitoylphosphatidylcholine (SPPC), palmitoyloleoylphosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), dioleoylphosphatidylglycerol (DOPG), dimyristoylphosphatidylglycerol (DMPG), distearoylphosphatidylglycerol (DSPG), dipalmitoylglycerophosphoglycerol (DPPG), dipalmitoylphosphatidylserine (DPPS), 1,2-dioleoyl-sn-glycero-3-phosphatidylserine (DOPS), dimyristoylphosphatidylserine (DMPS), distearoylphosphatidylserine (DSPS), dipalmitoylphosphatidic acid (DPPA), 1,2-dioleoyl-sn-glycero-3-phosphatidic acid (DOPA), dimyristoylphosphatidic acid (DMPA), distearoylphosphatidic acid (DSPA), dipalmitoylphosphatidylinositol (DPPI), 1,2-dioleoyl-sn-glycero-3-phosphatidylinositol (DOPI), dimyristoylphosphatidylinositol (DMPI), distearoylphosphatidylinositol (DSPI), and derivatives thereof; more preferably dipalmitoylphosphatidylcholine (DPPC), distearoylphosphatidylcholine (DSPC), dioleoylphosphatidylcholine (DOPC), dierucoylphosphatidylcholine (DEPC), dilauroylphosphatidylcholine (DLPC), hydrogenated soy phosphatidylcholine (HSPC), distearoylphosphatidylethanolamine (DSPE), and dimyristoylphosphatidylcholine (DMPC), and polyethylene glycol-modified derivatives thereof; most preferably hydrogenated soy phosphatidylcholine (HSPC) and polyethylene glycol 2000-distearoylphosphatidylethanolamine (mPEG2000-DSPE).

4. The pharmaceutical composition according to any one of claims 1-3, wherein the phospholipid has a molar percentage selected from the group consisting of 10%-80%, preferably 30%-70%, relative to a total amount of a lipid mixture.

5. The pharmaceutical composition according to any one of claims 1-4, wherein the lipid further comprises a steroid, preferably cholesterol.

6. The pharmaceutical composition according to claim 5, wherein the steroid has a molar percentage selected from the group consisting of 0.1%-90%, preferably 10%-80%, and more preferably 20%-70%, relative to the total amount of the lipid mixture.

7. The pharmaceutical composition according to any one of claims 1-6, further comprising a trapping agent, wherein the trapping agent is preferably a sulfate, a sulfite, a gluconate, a phosphate, a hydrogen phosphate, a molybdate, a carbonate, and a nitrate, and is more preferably ammonium sulfate, ammonium phosphate, ammonium molybdate, ammonium sucrose octasulfate, triethylammonium sucrose octasulfate, copper gluconate, ethylammonium sulfate, ammonium ethylenediaminetetraacetate, ammonium chloride, ammonium hydroxide, ammonium acetate, dextran ammonium sulfate, or triethyldextran ammonium sulfate, and combinations thereof.

8. The pharmaceutical composition according to any one of claims 1-7, wherein an inner aqueous phase of the liposome further comprises at least one pH adjuster, and the pH adjuster is preferably sodium hydroxide, hydrochloric acid, ammonia water, phosphoric acid, malic acid, histidine, or triethanolamine.

9. The pharmaceutical composition according to any one of claims 1-8, wherein the compound of formula I or the pharmaceutically acceptable salt thereof has a concentration selected from the group consisting of 0.01-100 mg/mL, preferably 0.05-10 mg/mL.

10. The pharmaceutical composition according to any one of claims 1-9, wherein an outer aqueous phase of the liposome comprises one or more selected from the group consisting of a sugar, an electrolyte, and an amino acid or a salt thereof, preferably sodium chloride and histidine or a salt thereof.

11. A lyophilized composition, wherein the lyophilized composition is obtained by lyophilizing or spray-drying the pharmaceutical composition according to any one of claims 1-10.

12. A lyophilized composition, wherein the lyophilized composition is capable of being reconstituted to give the pharmaceutical composition according to any one of claims **1-**10.

**13.** A reconstituted solution, wherein the reconstituted solution is obtained by reconstituting the lyophilized composition according to claim 11.

**14.** A liposome, comprising a compound of formula I or a pharmaceutically acceptable salt thereof,

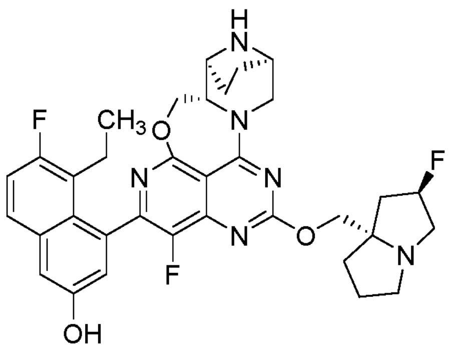

(I)

.

**15.** A method for preparing the pharmaceutical composition according to any one of claims 2-10 or the liposome according to claim 14, comprising steps of preparing a blank liposome, and introducing the compound of formula I or the pharmaceutically acceptable salt thereof into an inner aqueous phase of the blank liposome.

**16.** Use of the pharmaceutical composition according to any one of claims 1-10, the lyophilized composition according to claim 11 or 12 or the reconstituted solution according to claim 13 or the liposome according to claim 14 in the preparation of a medicament for treating a disease or disorder, wherein the disease or disorder is preferably a cancer, and is more preferably selected from the group consisting of brain cancer, thyroid cancer, head and neck cancer, nasopharyngeal cancer, laryngeal cancer, oral cancer, salivary gland cancer, esophageal cancer, gastric cancer, lung cancer, liver cancer, renal cancer, pancreatic cancer, gallbladder cancer, bile duct cancer, colorectal cancer, small intestine cancer, gastrointestinal stromal tumor, urothelial cancer, urinary tract cancer, bladder cancer, breast cancer, vaginal cancer, ovarian cancer, endometrial cancer, cervical cancer, fallopian tube cancer, testicular cancer, prostate cancer, hemangioma, leukemia, lymphoma, myeloma, skin cancer, lipoma, bone cancer, soft tissue sarcoma, neurofibroma, neuroglioma, neuroblastoma, and glioblastoma.

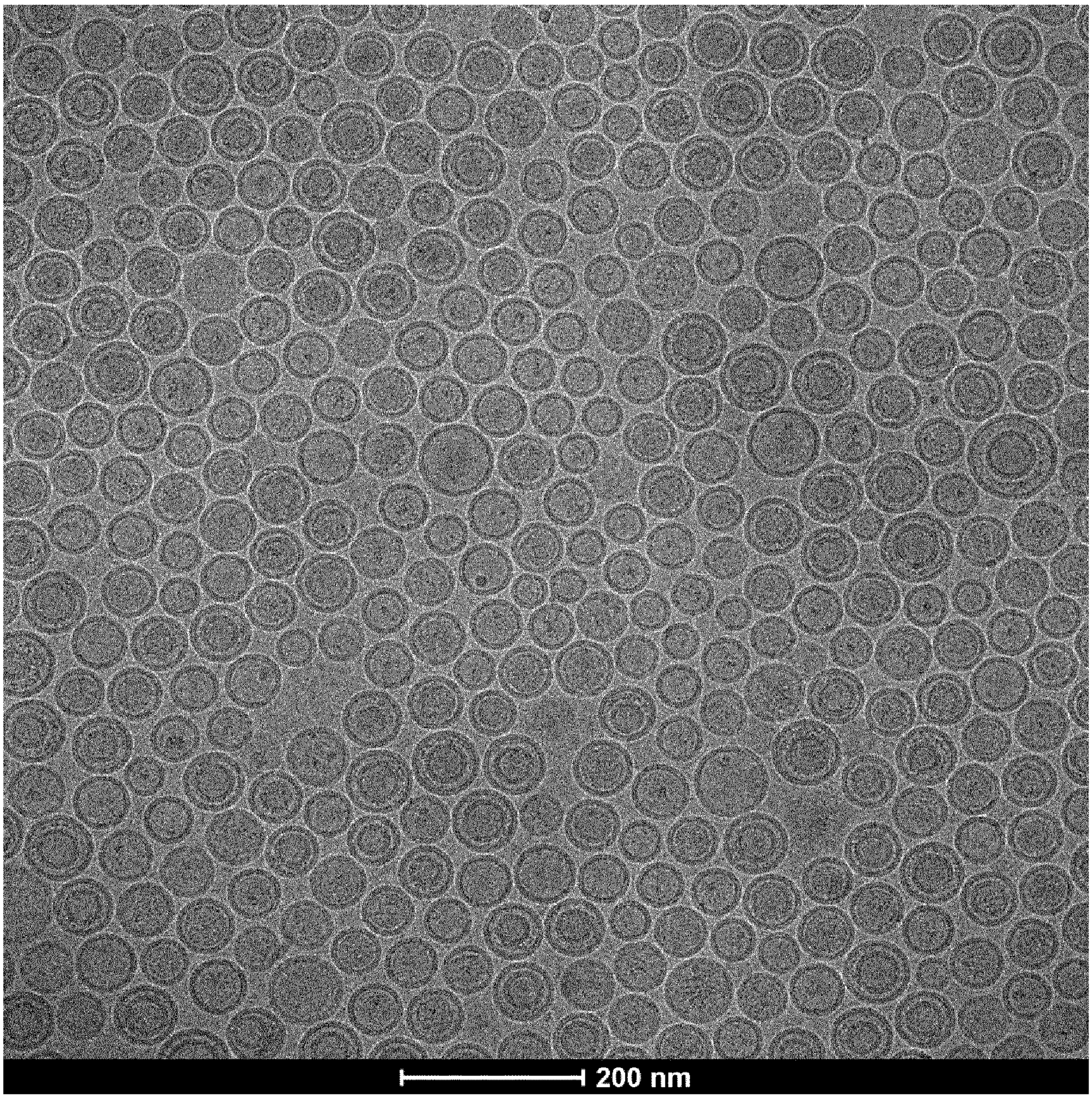


FIG. 1

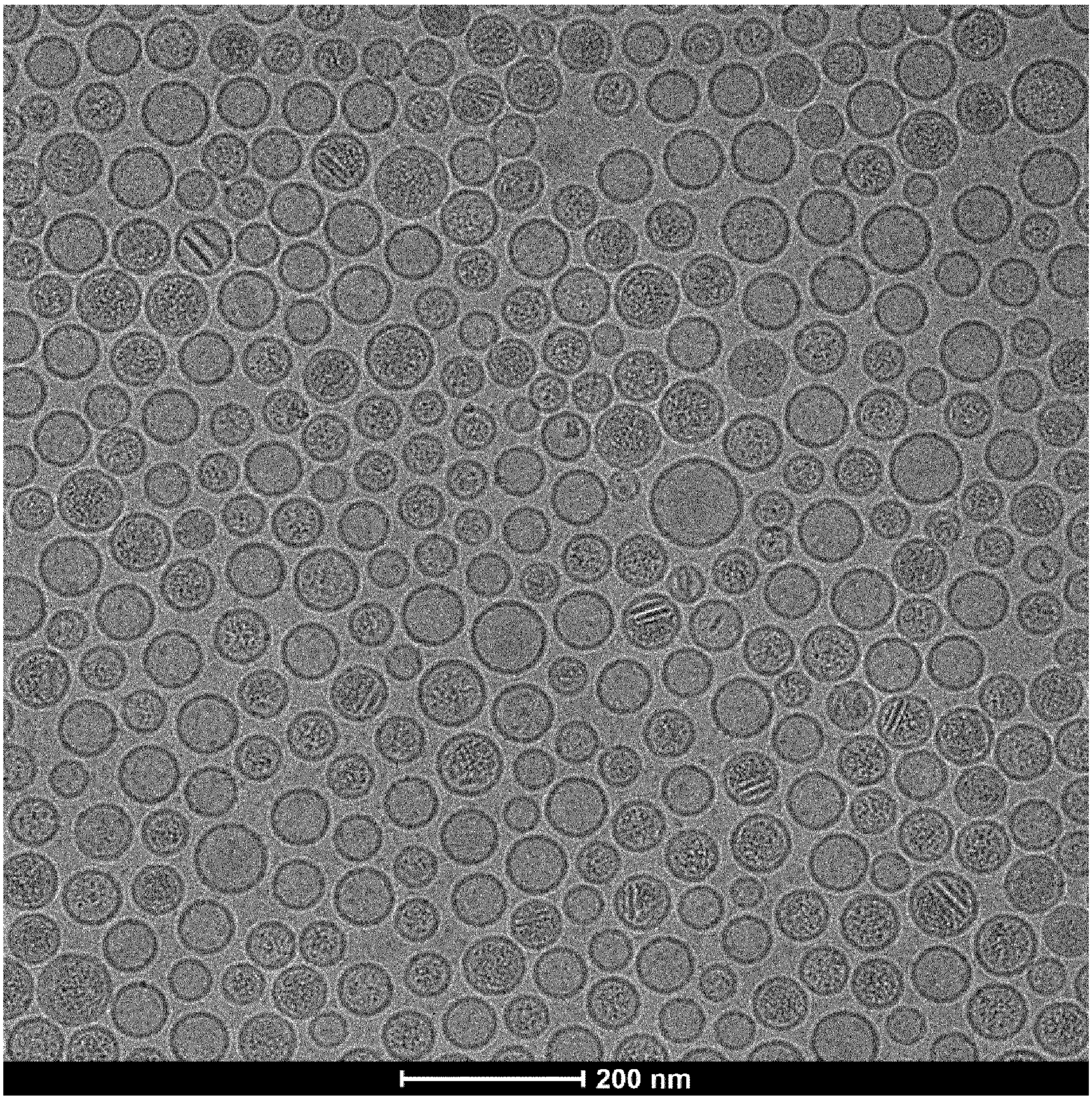


FIG. 2

**INTERNATIONAL SEARCH REPORT**

International application No.
**PCT/CN2023/109901**

**A. CLASSIFICATION OF SUBJECT MATTER**

A61K31/553(2006.01)i; A61K9/127(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNTXT, WPABS, WPABSC, DWPI, VEN, ENTXT, ENTXTC, CJFD, PUBMED, ISI-WEB OF SCIENCE, REGISTRY, CAPLUS: 式I结构式检索, structure search of formula (I), 脂质体, structure search of formula (I), liposome

**C. DOCUMENTS CONSIDERED TO BE RELEVANT**

| Category* | Citation of document, with indication, where appropriate, of the relevant passages | Relevant to claim No. |
|---|---|---|
| PX | WO 2022268051 A1 (JIANGSU HENGRUI PHARMACEUTICALS CO., LTD. et al.) 29 December 2022 (2022-12-29)<br>claims 1-22 | 1-16 |
| PX | WO 2023001141 A1 (SHANGHAI ZION PHARMA CO., LTD.) 26 January 2023 (2023-01-26)<br>claims 1-97 | 1-16 |
| PX | WO 2023001123 A1 (SHANGHAI ALLIST PHARMACEUTICALS CO., LTD.) 26 January 2023 (2023-01-26)<br>claims 1-21 | 1-16 |
| PX | WO 2022188729 A1 (JACOBIO PHARMACEUTICALS CO., LTD.) 15 September 2022 (2022-09-15)<br>claims 1-34 | 1-16 |
| PX | WO 2022194245 A1 (GENFLEET THERAPEUTICS SHANGHAI INC. et al.) 22 September 2022 (2022-09-22)<br>claims 1-15 | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 September 2023** | **05 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

INTERNATIONAL SEARCH REPORT

International application No.
**PCT/CN2023/109901**

**C. DOCUMENTS CONSIDERED TO BE RELEVANT**

| Category* | Citation of document, with indication, where appropriate, of the relevant passages | Relevant to claim No. |
|---|---|---|
| PX | Robert B. Kargbo. "Targeting KRASG12D Mutations: Discovery of Small Molecule Inhibitors for the Potential Treatment of Intractable Cancers" *ACS Med. Chem. Lett.*, Vol. 14, 11 July 2023 (2023-07-11), pages 1041-1042 abstract | 1-16 |
| A | CN 110603258 A (ASTRAZENECA AB) 20 December 2019 (2019-12-20) claims 1-16 | 1-16 |
| A | WO 2022017339 A1 (JIANGSU HENGRUI MEDICINE CO., LTD. et al.) 27 January 2022 (2022-01-27) claims 1-21 | 1-16 |
| A | WO 2021203768 A1 (JIANGSU HENGRUI MEDICINE CO., LTD. et al.) 14 October 2021 (2021-10-14) claims 1-22 | 1-16 |
| A | WO 2021107160 A1 (TAIHO PHARMACEUTICAL CO., LTD. et al.) 03 June 2021 (2021-06-03) claims 1-39 | 1-16 |

Form PCT/ISA/210 (second sheet) (July 2022)

**INTERNATIONAL SEARCH REPORT**
**Information on patent family members**

International application No.
**PCT/CN2023/109901**

| Patent document cited in search report | | | Publication date (day/month/year) | Patent family member(s) | | | Publication date (day/month/year) |
|---|---|---|---|---|---|---|---|
| WO | 2022268051 | A1 | 29 December 2022 | TW | 202317587 | A | 01 May 2023 |
| WO | 2023001141 | A1 | 26 January 2023 | | None | | |
| WO | 2023001123 | A1 | 26 January 2023 | | None | | |
| WO | 2022188729 | A1 | 15 September 2022 | | None | | |
| WO | 2022194245 | A1 | 22 September 2022 | | None | | |
| CN | 110603258 | A | 20 December 2019 | CA | 3061650 | A1 | 15 November 2018 |
| | | | | US | 2020109153 | A1 | 09 April 2020 |
| | | | | JP | 2020519589 | A | 02 July 2020 |
| | | | | EP | 3621968 | A1 | 18 March 2020 |
| | | | | AR | 111776 | A1 | 21 August 2019 |
| | | | | TW | 201906848 | A | 16 February 2019 |
| | | | | WO | 2018206539 | A1 | 15 November 2018 |
| | | | | US | 2022127281 | A1 | 28 April 2022 |
| WO | 2022017339 | A1 | 27 January 2022 | TW | 202214608 | A | 16 April 2022 |
| WO | 2021203768 | A1 | 14 October 2021 | TW | 202144338 | A | 01 December 2021 |
| | | | | CN | 115244058 | A | 25 October 2022 |
| WO | 2021107160 | A1 | 03 June 2021 | EP | 4065583 | A1 | 05 October 2022 |
| | | | | TW | 202134243 | A | 16 September 2021 |
| | | | | WO | 2021106231 | A1 | 03 June 2021 |
| | | | | JP | 2023512113 | A | 23 March 2023 |
| | | | | CN | 115003677 | A | 02 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022268051 A
- CN 2022100016 W
- WO 2021041671 A